# EUROPEAN PATENT APPLICATION

(11) **EP 4 488 304 A1**
(43) Date of publication of application: **08.01.2025**
(21) Application number: 23780081.8
(22) Date of filing: 23.03.2023
(51) Int. Cl.: C08F 290/06, A61C 13/01, A61C 13/20, B29C 64/124, B33Y 70/00, B33Y 80/00

(54) **PHOTOSETTING COMPOSITION, THREE-DIMENSIONALLY SHAPED ARTICLE, MOLD, METHOD FOR MANUFACTURING CURED PRODUCT, AND METHOD FOR MANUFACTURING PLATE DENTURE**

(30) Priority: 28.03.2022 JP 2022052228; 08.03.2023 JP 2023036051
(71) Applicant: Mitsui Chemicals, Inc., Tokyo 104-0028 (JP)
(72) Inventor: ENDO, Suguru, Sodegaura-shi, Chiba 299-0265 (JP); SAKAMAKI, Toshikazu, Sodegaura-shi, Chiba 299-0265 (JP); KIMURA, Mai, Sodegaura-shi, Chiba 299-0265 (JP); MURAI, Hiroki, Sodegaura-shi, Chiba 299-0265 (JP); HAYASHI, Takaaki, Sodegaura-shi, Chiba 299-0265 (JP); KOBAYASHI, Eiji, Sodegaura-shi, Chiba 299-0265 (JP)
(74) Representative: Hoffmann Eitle
(86) International application number: PCT/JP2023/011620
(87) International publication number: WO 2023/190071

(57) **Abstract**

A photocurable composition, contains a (meth)acrylic monomer component, and photopolymerization initiator, in a case in which a rectangular sheet-like test piece A1 with a length of 40 mm, a width of 10 mm, and a thickness of 1.0 mm is produced by photomodeling under conditions in which the photocurable composition is irradiated with visible light having a wavelength of 405 nm at an irradiation dose of 11 mJ/cm² to form a cured layer A1 with a thickness of 50 µm, the cured layer A1 is stacked in a thickness direction thereof to form a rectangular sheet-like modeling product A1 with a length of 40 mm, a width of 10 mm, and a thickness of 1.0 mm, and the modeling product A1 is irradiated with ultraviolet rays having a wavelength of 365 nm at an irradiation dose of 3 J/cm² to produce the test piece A1, a storage elastic modulus at 25 °C of the test piece A1 is 10 MPa or more and a storage elastic modulus at 37 °C of the test piece A1 is 400 MPa or less.

## Description

### Technical Field

The present disclosure relates to a photocurable composition, a three-dimensional modeling product, a mold, a method of producing a cured product, and a method of producing a plate denture.

### Background Art

Dental products such as dental prostheses and instruments for intraoral use have been studied in recent years. For example, in terms of the efficiency of modeling these dental products, methods of producing a three-dimensional modeling product such as a dental product by photomodeling using a 3D printer have been known (see, for example, Patent Document 1).

Patent Document 1: Japanese Patent No. 4160311

### SUMMARY OF INVENTION

### Technical Problem

Meanwhile, there have been a conventional method of producing a dental prosthesis, a plate denture, or the like, without using photomodeling with a 3D printer or the like.

With this method, for example, in a case in which producing a plate denture, first a mold of the patient's mouth is taken directly from the patient using an impression material, and a model that mimics the inside of the patient's mouth is made by referring to the impression material. A mold is then made from this model using silicone. Using the made mold as a mold, a curable composition can be injected into it and the plate denture can be produced by room temperature polymerization.

As described above, the conventional method that does not use photomodeling have the problem of complicated processes such as producing a model that mimics the inside of the patient's mouth and making a mold with silicone.

In contrast, the above-mentioned complication is eliminated by the method of directly producing a plate denture by photomodeling using a 3D printer. However, compared with the conventional method not using a 3D printer, the materials that can be properly cured by photomodeling are very limited, so the characteristics of the resulting plate denture are also limited. For example, the conventional method uses room temperature polymerization using a mold, but this method allows the use of many materials that can improve aesthetics and physical properties. In contrast, the materials used in such room temperature polymerization are often not usable in photomodeling with a 3D printer. As a result, it is difficult to adjust the desired properties of the resulting plate denture when photomodeling with a 3D printer.

As described above, there is a need for a method of producing a plate denture that allows a plate denture to be produced simply and that allows easy adjustment to obtain desired properties.

In order to achieve the above object, the inventors have discovered a method of producing a mold for producing a plate denture by photomodeling with a 3D printer, and using the mold to produce a plate denture.

On the other hand, in case in which a mold of a plate denture is produced from a photocurable composition and then used to produce a plate denture, depending on the type of polymerizable composition used to produce the plate denture, the mold itself may shrink and deform when the polymerizable composition poured into the mold is polymerized during the production of the plate denture, which may result in deformation of the resulting plate denture.

When removing a plate denture obtained by polymerizing a polymerizable composition in a mold from the mold, the plate denture may become stuck in the mold due to the fact that it contains artificial teeth, making it physically difficult to remove.

An object of one aspect of the present disclosure is to provide a photocurable composition capable of producing a mold that is suppressed from deforming during the production of a plate denture, or a mold that allows a plate denture to be easily removed from the mold when a denture is produced using the mold, and a three-dimensional modeling product, a mold, a method of producing a cured product, and a method of producing a plate denture which use this photocurable composition.

An object of another aspect of the present disclosure is to provide a method of producing a plate denture capable of producing a plate denture in a simple manner.

### Solution to Problem

Means for solving the above-described problems include the following aspects.
<1> A photocurable composition, comprising a (meth)acrylic monomer component, and a photopolymerization initiator, wherein:
   in a case in which a rectangular sheet-like test piece A1 with a length of 40 mm, a width of 10 mm, and a thickness of 1.0 mm is produced by photomodeling under conditions in which the photocurable composition is irradiated with visible light having a wavelength of 405 nm at an irradiation dose of 11 mJ/cm² to form a cured layer A1 with a thickness of 50 µm, the cured layer A1 is stacked in a thickness direction thereof to form a rectangular sheet-like modeling product A1 with a length of 40 mm, a width of 10 mm, and a thickness of 1.0 mm, and the modeling product A1 is irradiated with ultraviolet rays having a wavelength of 365 nm at an irradiation dose of 3 J/cm² to produce the test piece A1,
   a storage elastic modulus at 25°C of the test piece A1 is 10 MPa or more and
   a storage elastic modulus at 37°C of the test piece A1 is 400 MPa or less.
<2> The photocurable composition <1>, wherein the (meth)acrylic monomer component comprises:
   a mono(meth)acrylic monomer (X) having one (meth)acryloyloxy group and an aromatic ring,
   a di(meth)acrylic monomer (Y) having at least one of a cyclic structure or a urethane bond, and two (meth)acryloyloxy groups and not having a siloxane bond, and
   a polyfunctional (meth)acrylic monomer (Z) having a siloxane bond and two or more (meth)acryloyloxy groups.
<3> A photocurable composition, comprising a (meth)acrylic monomer component, and a photopolymerization initiator,
   wherein the (meth)acrylic monomer component comprises:
   a mono(meth)acrylic monomer (X) having one (meth)acryloyloxy group and an aromatic ring,
   a di(meth)acrylic monomer (Y) having at least one of a cyclic structure or a urethane bond, and two (meth)acryloyloxy groups and not having a siloxane bond, and
   a polyfunctional (meth)acrylic monomer (Z) having a siloxane bond and two or more (meth)acryloyloxy groups.
<4> The photocurable composition according to <2> or <3>, wherein a molecular weight of the di(meth)acrylic monomer (Y) is from 400 to 5000.
<5> The photocurable composition according to any one of <2> to <4>, wherein a molecular weight of the polyfunctional (meth)acrylic monomer (Z) is from 400 to 5000.
<6> The photocurable composition according to any one of <2> to <5>, wherein a content of the mono(meth)acrylic monomer (X) is from 30% by mass to 90% by mass with respect to a total amount of the (meth)acrylic monomer component.
<7> The photocurable composition according to any one of <2> to <6>, wherein a content of the di(meth)acrylic monomer (Y) is from 5% by mass to 55% by mass with respect to a total amount of the (meth)acrylic monomer component.
<8> The photocurable composition according to any one of <2> to <7>, wherein a content of the polyfunctional (meth)acrylic monomer (Z) is from 1% by mass to 60% by mass with respect to a total amount of the (meth)acrylic monomer component.
<9> The photocurable composition according to any one of <2> to <8>, wherein a siloxane bond concentration in the composition is from 0.100 mmol/g to 3.000 mmol/g.
<10> The photocurable composition according to any one of <1> to <9>, wherein an aromatic ring concentration in the (meth)acrylic monomer component is from 0.0015 mmol/g to 0.0070 mmol/g.
<11> The photocurable composition according to any one of <1> to <9>, wherein an aromatic ring concentration in the (meth)acrylic monomer component is from 0.0015 mmol/g to 0.0042 mmol/g.
<12> The photocurable composition according to any one of <1> to <11>, wherein the (meth)acrylic monomer component comprises di(meth)acrylic monomer (A) which has two (meth)acryloyloxy groups and an aromatic ring, and has a distance of from 25 Å to 80 Å between an oxygen atom forming an oxy group in one of the (meth)acryloyloxy groups and an oxygen atom forming an oxy group in another of the (meth)acryloyloxy groups.
<13> The photocurable composition according to any one of <1> to <12>, satisfying either of the following (a) or (b):
   (a) the (meth)acrylic monomer component comprises two or more types of di(meth)acrylic monomers (A) which have two (meth)acryloyloxy groups and an aromatic ring, and have a distance of from 25 Å to 80 Å between an oxygen atom forming an oxy group in one of the (meth)acryloyloxy groups and an oxygen atom forming an oxy group in another of the (meth)acryloyloxy groups,
   (b) the (meth)acrylic monomer component comprises one or more (meth)acrylic monomer (B) selected from the group consisting of:
      a di(meth)acrylic monomer (A) which has two (meth)acryloyloxy groups and an aromatic ring, and has a distance of from 25 Å to 80 Å between an oxygen atom forming an oxy group in one of the (meth)acryloyloxy groups and an oxygen atom forming an oxy group in another of the (meth)acryloyloxy groups;
      a di(meth)acrylic monomer (B-1) which has two (meth)acryloyloxy groups and at least one of an aromatic ring or a urethane bond, and has a distance of 10 Å or more and less than 25 Å between an oxygen atom forming an oxy group in one of the (meth)acryloyloxy groups and an oxygen atom forming an oxy group in another of the (meth)acryloyloxy groups;
      a di(meth)acrylic monomer (B-2) which has two (meth)acryloyloxy groups and at least one of an aromatic ring or a urethane bond, and has a distance of more than 80 Å and less than 200 Å between an oxygen atom forming an oxy group in one of the (meth)acryloyloxy groups and an oxygen atom forming an oxy group in another of the (meth)acryloyloxy groups; and
      a mono(meth)acrylic monomer (B-3) which has one (meth)acryloyloxy group and at least one of an aromatic ring or a hydroxy group.
<14> The photocurable composition according to <13>, satisfying the (b), and;
   in a case in which the (meth)acrylic monomer (B) comprises the di(meth)acrylic monomer (B-1), a molecular weight of the di(meth)acrylic monomer (B-1) is from 400 to 800,
   in a case in which the (meth)acrylic monomer (B) comprises the di(meth)acrylic monomer (B-2), a molecular weight of the di(meth)acrylic monomer (B-2) is from 900 to 3000,
   in a case in which the (meth)acrylic monomer (B) comprises the mono(meth)acrylic monomer (B-3), a molecular weight of the mono(meth)acrylic monomer (B-3) is from 130 to 350.
<15> The photocurable composition according to <13> or <14>, satisfying the (b), and;
   a content of the (meth)acrylic monomer (B) is from 3% by mass to 80% by mass with respect to a total amount of the (meth)acrylic monomer component.
<16> The photocurable composition according to any one of <12> to <15>, wherein a molecular weight of the di(meth)acrylic monomer (A) is from 650 to 1300.
<17> The photocurable composition according to any one of <12> to <16>, wherein a content of the di(meth)acrylic monomer (A) is 30% by mass or more with respect to a total amount of the (meth)acrylic monomer component.
<18> The photocurable composition according to any one of <1> to <17>, having a viscosity, which is measured by an E-type viscometer under conditions of 25°C and 50 rpm, of from 5 mPa·s to 6,000 mPa·s.
<19> The photocurable composition according to any one of <1> to <18>, which is a photocurable composition for photomodeling.
<20> The photocurable composition according to any one of <1> to <19>, which is a photocurable composition used in production of a mold by photomodeling.
<21> A three-dimensional modeling product, comprising a cured product of the photocurable composition according to any one of <1> to <20>.
<22> A mold, comprising the three-dimensional modeling product according to <21>.
<23> The mold according to <22>, which is a mold used in production of a plate denture.
<24> A method of producing a cured product, comprising a step of polymerizing a curable composition in the mold according to <22> or <23>.
<25> A method of producing a plate denture, comprising a step of curing a photocurable composition by photomodeling to produce a mold used in a production of a plate denture, and
   a step of polymerizing a curable composition in the mold to produce a plate denture.

### Advantageous Effects of Invention

According to one aspect of the present disclosure, a photocurable composition capable of producing a mold that is suppressed from deforming during the production of a plate denture, or a mold that allows a plate denture to be easily removed from the mold when a denture is produced using the mold, and a three-dimensional modeling product, a mold, a method of producing a cured product, and a method of producing a plate denture which use this photocurable composition can be provided.

According to another aspect of the present disclosure, a method of producing a plate denture capable of producing a plate denture in a simple manner.

### BRIEF DESCRIPTION OF DRAWING

FIG. 1 is a schematic diagram of a three-dimensional modeling product A2 formed in Examples.
FIG. 2 is a schematic diagram of a three-dimensional modeling product A3 formed in Examples.
FIG. 3 is a schematic diagram of a three-dimensional modeling product A4 formed in Examples.

### DESCRIPTION OF EMBODIMENTS

In the present disclosure, those numerical ranges that are expressed with "to" each denote a range that includes the numerical values stated before and after "to" as the lower limit value and the upper limit value, respectively.

In the present disclosure, when there are plural substances that correspond to a component of a composition, the indicated amount of the component in the composition means, unless otherwise specified, a total amount of the plural substances existing in the composition.

In a set of numerical ranges that are stated in a stepwise manner in the present disclosure, the upper limit value or the lower limit value of one numerical range may be replaced with the upper limit value or the lower limit value of other numerical range. Further, in a numerical range stated in the present disclosure, the upper limit value or the lower limit value of the numerical range may be replaced with a relevant value indicated in any of Examples.

In the present disclosure, "light" is a concept that encompasses active energy rays such as ultraviolet rays and visible light beams.

In the present disclosure, "(meth)acrylate" refers to an acrylate or a methacrylate, "(meth)acryloyl" refers to acryloyl or methacryloyl, and "(meth)acryl" refers to acryl or methacryl.

Hereinafter, a photocurable composition of the present disclosure will be described in terms of the first and second embodiments. The preferred forms of the first embodiment and the preferred forms of the second embodiment may be combined as appropriate. Unless otherwise specified, the preferred uses and physical properties of the photocurable composition of the second embodiment are the same as those of the photocurable composition of the first embodiment.

### [First embodiment]

### [Photocurable composition]

A photocurable composition of the first embodiment of the present disclosure contains a (meth)acrylic monomer component, and a photopolymerization initiator,
in a case in which a rectangular sheet-like test piece A1 with a length of 40 mm, a width of 10 mm, and a thickness of 1.0 mm is produced by photomodeling under conditions in which the photocurable composition is irradiated with visible light having a wavelength of 405 nm at an irradiation dose of 11 mJ/cm² to form a cured layer A1 with a thickness of 50 µm, the cured layer A1 is stacked in a thickness direction thereof to form a rectangular sheet-like modeling product A1 with a length of 40 mm, a width of 10 mm, and a thickness of 1.0 mm, and the modeling product A1 is irradiated with ultraviolet rays having a wavelength of 365 nm at an irradiation dose of 3 J/cm² to produce the test piece A1,
a storage elastic modulus at 25°C of the test piece A1 is 10 MPa or more and
a storage elastic modulus at 37°C of the test piece A1 is 400 MPa or less.

The photocurable composition of the present disclosure contains a (meth)acrylic monomer component, and a photopolymerization initiator, the test piece A1 produced by the conditions described above has the storage elastic modulus at 25°C of 10 MPa or more and the storage elastic modulus at 37°C of 400 MPa or less. A mold that is suppressed from deforming during the production of a plate denture, or a mold that allows a plate denture to be easily removed from the mold when the plate denture is produced using the mold can be produced by using such a photocurable composition.

The photocurable composition of the present disclosure is a composition that cures when irradiated with light, and a cured product is obtained by curing this composition. Photomodeling is preferable as a method of producing a cured product using the photocurable composition of the present disclosure.

The photocurable composition of the present disclosure is preferably a photocurable composition for photomodeling, in other words, a cured product produced using the photocurable composition of the present disclosure is preferably a photofabrication product (i.e., a cured product by photomodeling).

Photomodeling is a method in which a photocurable composition is irradiated with light to form a cured layer, and the process is repeated to stack the cured layer to obtain a cured product (i.e., a photofabrication product).

Photomodeling may be inkjet photomodeling or liquid vat photomodeling (i.e., photomodeling using a vat).

In inkjet photomodeling, droplets of a photocurable composition are ejected from an inkjet nozzle onto a substrate, and the droplets attached to the substrate are irradiated with light to obtain a cured product.

In one example of inkjet photomodeling, for example, a head equipped with an inkjet nozzle and a light source is scanned in a plane while a photocurable composition is ejected from an inkjet nozzle onto a substrate, and the ejected photocurable composition is irradiated with light to form a cured layer. By repeating these operations, cured layers are successively stacked to obtain a cured product (i.e., a photofabrication product).

In vat photomodeling, a photocurable composition (i.e., an uncured photocurable composition in a liquid state; the same applies below) housed in a vat is partially cured by photoirradiation to form a cured layer, and the cured layer is disposed on one another by repeating this operation, whereby a three-dimensional modeling product is obtained. Vat photomodeling is different from inkjet photomodeling in that it uses a vat.

Vat photomodeling is broadly classified into DLP (Digital Light Processing) photomodeling and SLA (Stereolithography) photomodeling.

In DLP photomodeling, a photocurable composition in a vat is irradiated with planar light.

In SLA photomodeling, laser light is scanned over a photocurable composition in a vat.

From the viewpoint of more effectively exerting the effects of the photocurable composition of the present disclosure, DLP photomodeling is preferable as vat photomodeling.

In one example of DLP photomodeling, for example, a 3D printer (e.g., "CARA PRINT 4.0" manufactured by Kulzer GmbH, or "MAX UV" manufactured by Asiga) that includes the followings is employed:
a vertically movable build table;
a tray (i.e., a vat) which is arranged below the build table (on the side of the gravity direction; the same applies below) and which includes a light transmitting section and houses a photocurable composition; and
a light source (e.g., an LED light source) which is arranged below the tray and used for irradiating the photocurable composition in the tray with planar light through the light transmitting section of the tray.

In this example, first, a gap equivalent to a single layer is created between the build table and the tray, and this gap is filled with a photocurable composition. Next, the photocurable composition filling the gap is irradiated with planar light from below through the light transmitting section of the tray to cure the light-irradiated region, whereby a first cured layer is formed. Subsequently, the gap between the build table and the tray is expanded for another layer to be formed next, and the resulting space is filled with the photocurable composition. Then, the photocurable composition filling the space is irradiated with light in the same manner as in the curing of the first layer to form a second cured layer. The above-described operations are repeated to dispose cured layers on one another, whereby a three-dimensional modeling product is produced. In this example, the thus produced three-dimensional modeling product may be further irradiated with light and thereby further cured.

Regarding DLP photomodeling, for example, the descriptions Japanese Patent No. 5111880 and Japanese Patent No. 5235056 may be referred.

### <Use>

The use of the photocurable composition of the present disclosure is not particularly limited, and for example, it is preferably a photocurable composition used in the production of a mold, a dental product, or the like by photomodeling, and more preferably a photocurable composition used in the production of a mold by photomodeling.

Examples of the mold include a mold used in the production of a plate denture. For example, a plate denture may be produced by injecting a curable composition for producing a denture base into a mold in which artificial teeth have been arranged, and then curing the curable composition after injection.

Examples of the dental product include a dental prosthesis, a medical instrument for intraoral use, a dental model, or a lost-foam casting model.

Examples of the dental prosthesis include an inlay, a crown, a bridge, a temporary crown, and a temporary bridge.

Examples of the medical instrument for intraoral use include a denture (for example, a complete denture, a partial dentures, or the like), a mouthpiece, a mouth guard, a orthodontic appliance, a bite splint, a splint for treating a temporomandibular joint disorder, an impression tray, and a surgical guide.

Examples of the dental model include a jaw model.

In the photocurable composition of the present disclosure, the storage elastic modulus at 25°C of the test piece A1 is 10 MPa or more and from the viewpoint of suppressing deformation of a mold (for example, from the viewpoint of suppressing deformation of a mold when a photocurable composition for producing denture base is injected into a mold produced by using the photocurable composition of the present disclosure and then polymerized), it is preferably 15 MPa or more, more preferably 30 MPa or more, and still more preferably 50 MPa or more.

The upper limit of the storage elastic modulus at 25°C of the test piece A1 is not particularly limited and for example, from the viewpoint of the removability from a mold (for example, from the viewpoint of suppressing damage to a mold, a plate denture, or the like, when the plate denture is removed from the mold), it may be 2000 MPa or less, may be 1000 MPa or less, or may be 500 MPa or less.

In the photocurable composition of the present disclosure, the storage elastic modulus at 37°C of the test piece A1 is 400 MPa or less and from the viewpoint of the removability from a mold, it is preferably 300 MPa or less, more preferably 250 MPa or less, and still more preferably 200 MPa or less.

The lower limit of the storage elastic modulus at 37°C of the test piece A1 is not particularly limited and for example, from the viewpoint of suppressing deformation of a mold when in use, it may be 10 MPa or more, may be 30 MPa or more, or may be 50 MPa or more.

In the photocurable composition of the present disclosure, the aromatic ring concentration in the (meth)acrylic monomer component is from 0.0015 mmol/g to 0.0042 mmol/g, more preferably from 0.0016 mmol/g to 0.0041 mmol/g, and still more preferably from 0.0017 mmol/g to 0.0040 mmol/g. By adjusting the aromatic ring concentration in the (meth)acrylic monomer component as described above, there is a tendency that the removability from a mold is excellent and deformation of a mold during use can be suitably suppressed.

From the viewpoint that the photocurable composition of the present disclosure is more excellent in the removability from a mold and can suitably suppress deformation of a mold during use, it is preferable to satisfy any of the following conditions (1) to (3).
(1) the aromatic ring concentration in the (meth)acrylic monomer component is from 0.0030 mmol/g to 0.0042 mmol/g.
(2) the aromatic ring concentration in the (meth)acrylic monomer component is from 0.0015 mmol/g to 0.0035 mmol/g, and the urethane bond concentration in the (meth)acrylic monomer component is from 0.0001 mmol/g to 0.0020 mmol/g.
(3) the aromatic ring concentration in the (meth)acrylic monomer component is from 0.0015 mmol/g to 0.0035 mmol/g, and the hydroxy group concentration in the (meth)acrylic monomer component is from 0.0005 mmol/g to 0.0030 mmol/g.

The urethane bond concentration in the (meth)acrylic monomer component may be from 0.0001 mmol/g to 0.0020 mmol/g as the aforementioned (2), may be from 0.0002 mmol/g to 0.0018 mmol/g, or may be from 0.0004 mmol/g to 0.0016 mmol/g

The hydroxy group concentration in the (meth)acrylic monomer component may be from 0.0005 mmol/g to 0.0030 mmol/g as the aforementioned (3), may be from 0.0006 mmol/g to 0.0025 mmol/g, or may be from 0.0008 mmol/g to 0.0022 mmol/g.

### <(Meth)acrylic monomer component>

The photocurable composition of the present disclosure contains at least one type of a (meth)acrylic monomer component.

From the viewpoint of mechanical strength of a cured product, the content of the (meth)acrylic monomer component with respect to the total amount of the photocurable composition of the present disclosure is preferably 60% by mass or more, more preferably 80% by mass or more, and still more preferably 90% by mass or more.

The upper limit of the content of the (meth)acrylic monomer component with respect to the total amount of the photocurable composition of the present disclosure is not particularly limited as long as it is less than 100% by mass, and for example, may be 99.9% by mass or less.

Herein, the (meth)acrylic monomer component means a monomer having one or more (meth)acryloyloxy group in a molecule.

The photocurable composition of the present disclosure may contain or may not contain a photopolymerizable component other than the (meth)acrylic monomer component.

Examples of the photopolymerizable component other than the (meth)acrylic monomer component include styrene, styrene derivatives, and (meth)acrylonitrile.

In the photocurable composition of the present disclosure, the content of the photopolymerizable component other than the (meth)acrylic monomer component may be 20% by mass or less, may be 10% by mass or less, may be 5% by mass or less, or may be 1.0% by mass or less, with respect to the total amount of the photopolymerizable component in the photocurable composition of the present disclosure.

The lower limit of the content of the photopolymerizable component other than the (meth)acrylic monomer component is not particularly limited, and for example, may be 0% by mass or more.

The (meth)acrylic monomer constituting the (meth)acrylic monomer component may be any monomer as long as it has one or more (meth)acryloyl groups in the molecule, and there is no other particular limitation.

The (meth)acrylic monomer may be a monofunctional (meth)acrylic monomer (i.e., a monomer having one (meth)acryloyl group in the molecule), a bifunctional (meth)acrylic monomer (i.e., a monomer having two (meth)acryloyl groups in the molecule), or a polyfunctional (meth)acrylic monomer (i.e., a monomer having three or more (meth)acryloyl groups in the molecule).

From the viewpoint of reducing the storage elastic modulus at 25°C and the storage elastic modulus at 37°C of the test piece A1, it is preferable that the (meth)acrylic monomer component preferably contains a di(meth)acrylic monomer (A) which has two (meth)acryloyloxy groups and an aromatic ring, and has a distance (hereinafter, also referred to as "distance d1") of from 25 Å to 80 Å between an oxygen atom forming an oxy group in one of the (meth)acryloyloxy groups and an oxygen atom forming an oxy group in another of the (meth)acryloyloxy groups.

The (meth)acrylic monomer may contain one type of di(meth)acrylic monomer (A) alone or two or more types of di(meth)acrylic monomer (A).

In the present disclosure, the d1 (i.e., the distance between an oxygen atom forming an oxy group in one of the (meth)acryloyloxy groups and an oxygen atom forming an oxy group in another of the (meth)acryloyloxy groups) refers to a linear distance between these two oxygen atoms.

The d1 means a value determined using the "Display Distance Measurement" function of "CHEM 3D" (version 18.2.0.48) manufactured by PerkinElmer Co., Ltd.

The d1 of the di(meth)acrylic monomer (A) is from 25 Å to 80 Å, for example, may be from 30 Å to 60 Å, or may be from 30 Å to 50 Å.

The di(meth)acrylic monomer (A) preferably contains a cyclic structure. Examples of the cyclic structure include an aromatic structure and an alicyclic structure. Among them, the di(meth)acrylic monomer (A) preferably contains an aromatic structure, and more preferably contains a bisphenol structure such as bisphenol A or bisphenol F.

The di(meth)acrylic monomer (A) may contain a total of one or more of at least one of an ethyleneoxy group or a propyleneoxy group.

The molecular weight of the di(meth)acrylic monomer (A) is preferably from 650 to 1300, more preferably from 700 to 1200, and still more preferably from 750 to 1000.

The weight average molecular weight of the di(meth)acrylic monomer (A) is preferably from 650 to 1300, more preferably from 700 to 1200, and still more preferably from 750 to 1000.

In the present disclosure, the weight average molecular weight is measured by gel permeation chromatography (GPC).

Examples of the di(meth)acrylic monomer (A) include ethoxylated bisphenol A di(meth)acrylate, propoxylated bisphenol A di(meth)acrylate, ethoxylated bisphenol F di(meth)acrylate, and propoxylated bisphenol F di(meth)acrylate.

The content of the di(meth)acrylic monomer (A) is preferably 30% by mass or more, more preferably from 40% by mass to 100% by mass, and still more preferably from 50% by mass to 100% by mass, with respect to the total amount of the (meth)acrylic monomer component.

From the viewpoint of ensuring the removability and easily forming a mold that is easy to suppress deformation, the (meth)acrylic monomer component preferably satisfies either of the following (a) or (b).
(a) the (meth)acrylic monomer component contains two or more types of the aforementioned di(meth) acrylic monomer (A).
(b) the (meth)acrylic monomer component contains one or more (meth)acrylic monomer (B) selected from the group consisting of:
   a di(meth)acrylic monomer (B-1) which has two (meth)acryloyloxy groups and at least one of an aromatic ring or a urethane bond, and has the distance d1 of 10 Å or more and less than 25 Å;
   a di(meth)acrylic monomer (B-2) which has two (meth)acryloyloxy groups and at least one of an aromatic ring or a urethane bond, and has the distance d1 of more than 80 Å and less than 200 Å; and
   a mono(meth)acrylic monomer (B-3) which has one (meth)acryloyloxy group and at least one of an aromatic ring or a hydroxy group.

In a case in which the (meth)acrylic monomer component satisfies the aforementioned (a), it is sufficient that the (meth)acrylic monomer component contains two or more types of di(meth)acrylic monomer (A), and the (meth)acrylic monomer component may or may not contain another (meth)acrylic monomer component.

The (meth)acrylic monomer component containing two or more types of di(meth)acrylic monomers (A) not only reduces the storage elastic modulus at 25°C and the storage elastic modulus at 37°C of the test piece A1, but also makes it easier to control the reactivity than in a case in which one type of di(meth)acrylic monomer (A) is used, resulting in excellent operability during photofabrication.

In a case in which the (meth)acrylic monomer component satisfies the aforementioned (a), the total content of the di(meth)acrylic monomer (A) is preferably from 50% by mass to 100% by mass, more preferably from 70% by mass to 100% by mass, and still more preferably from 90% by mass to 100% by mass, with respect to the total amount of the (meth)acrylic monomer component.

In a case in which the (meth)acrylic monomer component satisfies the aforementioned (b), the (meth)acrylic monomer component contains the aforementioned di(meth)acrylic monomer (A) and (meth)acrylic monomer (B). In this case, the (meth)acrylic monomer component may independently contain one type of the di(meth)acrylic monomer (A) and the (meth)acrylic monomer, or may contain two or more types of them.

Hereinafter, the details of the di(meth)acrylic monomer (B-1), di(meth)acrylic monomer (B-2), and mono(meth)acrylic monomer (B-3) which are classified as the (meth)acrylic monomer will be described.

### «Di(meth)acrylic monomer (B-1)»

The di(meth)acrylic monomer (B-1) is a (meth)acrylic monomer component which has two (meth)acryloyloxy groups and at least one of an aromatic ring or a urethane bond, and has the distance d1 of 10 Å or more and less than 25 Å. Use in the di(meth)acrylic monomer (B-1) tends to increase the storage elastic modulus at 25°C and the storage elastic modulus at 37°C of the test piece A1.

The d1 in di(meth)acrylic monomer (B-1) may be from 12 Å to 24 Å, or may be from 14 Å to 22 Å.

The molecular weight of the di(meth)acrylic monomer (B-1) is preferably from 400 to 800, more preferably from 400 to 700, and still more preferably from 400 to 650.

The weight average molecular weight of the di(meth)acrylic monomer (B-1) is preferably from 400 to 800, more preferably from 400 to 700, and still more preferably from 400 to 650.

The di(meth)acrylic monomer (B-1) has at least one of an aromatic ring or a urethane bond. The di(meth)acrylic monomer (B-1) may contain only one of an aromatic ring and a urethane bond, or may contain both an aromatic ring and a urethane bond.

In a case in which the di(meth)acrylic monomer (B-1) contain an aromatic ring and does not contain a urethane bond, the di(meth)acrylic monomer (B-1) may have at least one of an ethyleneoxy group or a propyleneoxy group, or the di(meth)acrylic monomer (B-1) may have a bisphenol structure and at least one of an ethyleneoxy group or a propyleneoxy group.

In a case in which the di(meth)acrylic monomer (B-1) contain an aromatic ring and does not contain a urethane bond, the specific examples of the di(meth)acrylic monomer (B-1) include an ethoxylated bisphenol A di(meth)acrylate, propoxylated bisphenol A di(meth)acrylate, ethoxylated bisphenol F di(meth)acrylate, and propoxylated bisphenol F di(meth)acrylate

When the di(meth)acrylic monomer (B-1) contain a urethane bond, it may contain a compound represented by the following Formula (1).

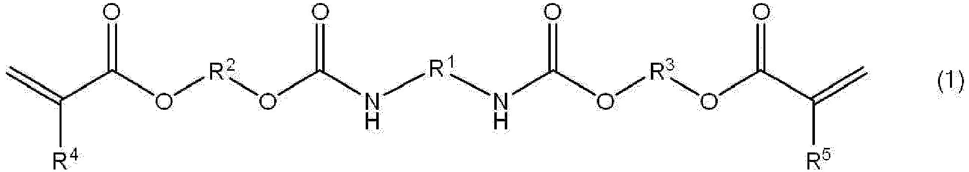

In Formula (1), R¹ is a divalent chain hydrocarbon group,
R² and R³ are each independently a divalent chain hydrocarbon group which may have a substituent,
R⁴ and R⁵ are each independently a methyl group or a hydrogen atom.

In R¹ in Formula (1), the number of carbon atoms in the divalent chain hydrocarbon group is preferably from 1 to 20, more preferably from 1 to 10, and still more preferably from 2 to 6.

The divalent chain hydrocarbon group in R¹ may be linear or branched, saturated or unsaturated, and may have a substituent.

The divalent chain hydrocarbon group in R¹ is preferably a linear or branched alkylene group having from 1 to 20 carbon atoms, more preferably a linear or branched alkylene group having from 1 to 12 carbon atoms, and still more preferably a linear or branched alkylene group having from 1 to 10 carbon atoms.

Specific examples of the linear or branched alkylene group having from 1 to 20 carbon atoms described above include methylene group, ethylene group, propanediyl group, butanediyl group, pentanediyl group, hexanediyl group, heptanediyl group, octanediyl group, nonanediyl group, decanediyl group, undecanediyl group, dodecanediyl group, tridecanediyl group, tetradecanediyl group, pentadecanediyl group, octadecanediyl group, eicosylene group, vinylene group, propenediyl group, butenediyl group, pentanediyl group, ethynylene group, propynylene group, and 2,4,4-trimethylhexylene group. Among these, hexanediyl group and 2,4,4-trimethylhexylene group are preferred.

In Formula (1), R² and R³ are each independently a divalent chain hydrocarbon group which may have a substituent.

The divalent chain hydrocarbon group suitable for R² and R³ is the same as the divalent chain hydrocarbon group suitable for R¹.

However, the number of carbon atoms in the divalent chain hydrocarbon group which may have a substituent for R² and R³ is preferably from 2 to 6, and more preferably from 2 to 3.

Examples of the substituent described above in a case in which R² and R³ are the divalent chain hydrocarbon group which have the substituent include;
an alkyl group having from 1 to 6 carbon atoms, such as a methyl group or an ethyl group;
an aryl group;
a cycloalkyl group having from 3 to 6 carbon atoms, such as a cyclopentyl group or a cyclohexyl group;
a tolyl group;
a xylyl group;
a cumyl group;
a styryl group;
an alkoxyphenyl group such as a methoxyphenyl group, an ethoxyphenyl group, or a propoxyphenyl group,
a phenoxyalkyl group such as a phenoxymethyl group, a phenoxyethyl group, or a phenoxypropyl group, and the like.

### «Di(meth)acrylic monomer (B-2)>>

A di(meth)acrylic monomer (B-2) is a (meth)acrylic monomer component which has two (meth)acryloyloxy groups and at least one of an aromatic ring or a urethane bond, and has the distance d1 of more than 80 Å and less than 200 Å. Use in the di(meth)acrylic monomer (B-2) tends to decrease the storage elastic modulus at 25°C and storage elastic modulus at 37°C of the test piece A1.

The d1 of the di(meth)acrylic monomer (B-2) may be from 85 Å to 150 Å, or may be from 90 Å to 120 Å.

The molecular weight of the di(meth)acrylic monomer (B-2) is preferably from 900 to 3000, more preferably from 1200 to 2500, and still more preferably from 1500 to 2000.

The weight average molecular weight of the di(meth)acrylic monomer (B-2) is preferably from 900 to 3000, more preferably from 1200 to 2500, and still more preferably from 1500 to 2000.

The di(meth)acrylic monomer (B-2) has at least one of an aromatic ring or a urethane bond. The di(meth)acrylic monomer (B-2) may contain only one of an aromatic ring and a urethane bond, or may contain both of an aromatic ring and a urethane bond.

In a case in which the di(meth)acrylic monomer (B-2) contain a urethane bond, it may contain a compound represented by Formula (2) described below.

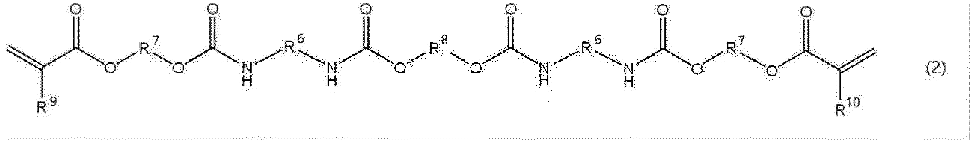

In Formula (2), R⁶'s are each independently a divalent chain hydrocarbon group, a divalent hydrocarbon group having an aromatic structure, or a divalent hydrocarbon group having an alicyclic structure,
R⁷'s is each independently a divalent chain hydrocarbon group which may have a substituent,
R₈ is a divalent linking group, and
R⁹ and R¹⁰ are each independently a methyl group or a hydrogen atom.

In Formula (2), in a case in which R⁶ is a divalent chain hydrocarbon group, the preferable constitution of R⁶ is the same as the preferable constitution of R¹ in Formula (1).

In R⁶ in Formula (2), the divalent hydrocarbon group having an aromatic structure is preferably a divalent hydrocarbon group having an aromatic structure having from 6 to 20 carbon atoms (more preferably from 6 to 12 carbon atoms, and still more preferably from 6 to 10 carbon atoms) which may have a substituent.

Examples of the divalent hydrocarbon group having an aromatic structure include an arylene group, an alkylenearylene group, an alkylenearylenealkylene group, and an arylenealkylenearylene group.

The divalent hydrocarbon group having an aromatic structure is preferably an alkylenearylene group or an alkylenearylenealkylene group.

Specific examples of the arylene group, alkylenearylene group, alkylenearylenealkylene group, alkylarylene group and arylenealkylenearylene group include a 1,3- or 1,4-phenylene group, a 1,3- or 1,4-phenylenedimethylene group, and a 1,3- or 1,4-phenylenediethylene group.

In R⁶ of Formula (2), the divalent hydrocarbon group having an alicyclic structure preferably has from 3 to 20 carbon atoms, more preferably from 6 to 12 carbon atoms, and still more preferably from 6 to 8 carbon atoms.

Examples of the alicyclic structures include cyclopropylene group, cyclobutylene group, cyclopentylene group, cyclohexylene group, cyclohexenylene group, cycloheptylene group, cyclooctylene group, cyclononylene group, cyclodecylene group, cycloundecylene group, cyclododecylene group, cyclotridecylene group, cyclotetradecylene group, cyclopentadecylene group, cyclooctadecylene group, cycloicosylene group, bicyclohexylene group, norbomylene group, isobomylene group, adamantylene group, and methylenebiscyclohexylene group.

The divalent hydrocarbon group having an alicyclic structure represented by R⁶ in Formula (2) may have a substituent. Examples of the substituent include a linear or branched alkyl group having from 1 to 6 carbon atoms.

In Formula (2), the preferable constitution of R⁷ is the same as the preferable constitutions of R² and R³ in Formula (1).

In Formula (2), R⁸ is a divalent linking group. Examples of the divalent linking group include a polyether group, an alkylene group, an arylene group, an alkylenearylene group, and an alkylenearylenealkylene group. Among them, a polyether group is preferable, and a polyether group composed of an ether group having from 2 to 4 carbon atoms is more preferable.

### «Mono(meth)acrylic monomer (B-3)>>

A mono(meth)acrylic monomer (B-3) is a (meth)acrylic monomer component having one (meth)acryloyloxy group and at least one of an aromatic ring or a hydroxy group. By using the mono(meth)acrylic monomer (B-3), there is a tendency that the storage elastic modulus at 25°C and the storage elastic modulus at 37°C of the test piece A1 increase while the viscosity of the photocurable composition decreases.

The molecular weight of the mono(meth)acrylic monomer (B-3) is preferably from 130 to 350, more preferably from 130 to 320, and still more preferably from 130 to 300.

The weight average molecular weight of the mono(meth)acrylic monomer (B-3) is preferably from 130 to 350, more preferably from 130 to 320, and still more preferably from 130 to 300.

The mono(meth)acrylic monomer (B-3) may contains a compound represented by the following Formula (3).

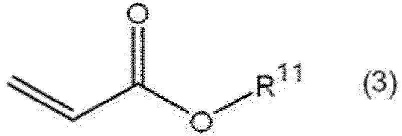

In Formula (3), R¹¹ is a monovalent organic group having at least one of an aromatic structure or a hydroxy group.

The monovalent organic group having an aromatic structure in R¹¹ in Formula (3) is preferably a monovalent organic group having from 2 to 30 carbon atoms, and more preferably a monovalent organic group having from 3 to 20 carbon atoms.

In Formula (3), R¹¹ may be an organic group represented by the following Formula (4).

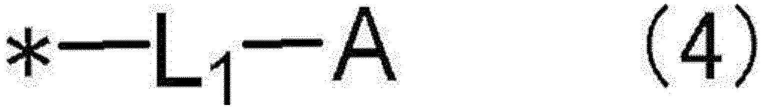

In Formula (4), L₁ is a single bond or a divalent chain hydrocarbon group having from 1 to 30 carbon atoms, which may have a heteroatom that is O or N. A is a hydroxyalkyl group having from 2 to 10 carbon atoms, or an aryl group having from 6 to 30 carbon atoms. * denotes a bonding position.

In Formula (4), the divalent chain hydrocarbon group represented by L₁ having from 1 to 30 carbon atoms, which may have a heteroatom that is O or N may be linear or branched.

The divalent chain hydrocarbon group represented by L₁ having from 1 to 30 carbon atoms, which may have a heteroatom that is O or N preferably has the number of carbon atoms from 1 to 20, more preferably from 1 to 10, and still more preferably from 1 to 8.

In a case in which the divalent chain hydrocarbon group represented by L₁ contains a heteroatom, the number of heteroatoms in L₁ is preferably from 1 to 3 and more preferably 1 or 2.

The divalent chain hydrocarbon group represented by L₁ above may have a substituent.

Suitable examples of the substituent include an alkyl group having from 1 to 3 carbon atoms, a hydroxy group, and an alkyl group having from 1 to 3 carbon atoms in which 1 or 2 of the hydrogen atoms are substituted with a hydroxy group.

The divalent chain hydrocarbon group represented by L₁ above may contain a urethane bond. In a case in which the divalent chain hydrocarbon group represented by L₁ above contains a urethane bond, the number of urethane bonds in L₁ may be 1 or 2.

Specific examples of the divalent chain hydrocarbon group represented by L₁ in Formula (4) above include the following groups. In the following groups, * denotes a bonding position.

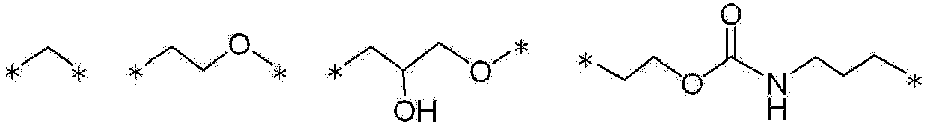

In Formula (4), in a case in which A is the hydroxyalkyl group having from 2 to 10 carbon atoms, L₁ is preferably a single bond. In this case, the mono(meth)acrylic monomer (B-3) is preferably 4-hydroxybutyl(meth)acrylate or 2-hydroxypropyl(meth)acrylate.

In Formula (4), examples of an aromatic structure in the aryl group represented by A having from 6 to 30 carbon atoms include a phenyl structure, a biphenyl structure, a naphthyl structure, and an anthryl structure.

The group represented by A in Formula (4) may have a substituent.

Suitable examples of the substituent above include;
an alkyl group having from 1 to 6 carbon atoms, such as a methyl group or an ethyl group;
a hydroxy group;
an alkyl group having from 1 to 6 carbon atoms, substituted with one or two hydroxy groups;
an aryl group;
a cycloalkyl group having from 3 to 6 carbon atoms, such as a cyclopentyl group or a cyclohexyl group;
a tolyl group;
a xylyl group;
a cumyl group;
a styryl group;
an alkoxyphenyl group such as a methoxyphenyl group, an ethoxyphenyl group, or a propoxyphenyl group,
a phenoxyalkyl group such as a phenoxymethyl group, a phenoxyethyl group, or a phenoxypropyl group, and the like.

Examples of the group represented by A in Formula (4) include the following examples. * denotes a bonding position.

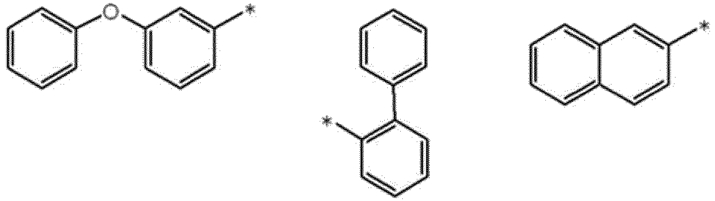

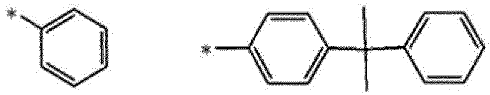

The content of the (meth)acrylic monomer (B) is preferably from 3% by mass to 80% by mass, more preferably from 10% by mass to 70% by mass, and still more preferably from 20% by mass to 60% by mass, with respect to the total amount of the (meth)acrylic monomer component.

The total content of the di(meth)acrylic monomer (A) and the (meth)acrylic monomer (B) is preferably 80% by mass or more, more preferably 90% by mass or more, and still more preferably 95% by mass or more, with respect to the total amount of the (meth)acrylic monomer component contained in the photocurable composition of the present disclosure.

The upper limit of the total content of the di(meth)acrylic monomer (A) and the (meth)acrylic monomer (B) is not particularly limited and may be 100% by mass or less.

In the photocurable composition of the present disclosure, the storage elastic modulus of the test piece A1 at 25°C can be improved preferentially over the storage elastic modulus of the test piece A1 at 37°C by increasing the content of the acrylic group among the methacrylic group and acrylic group of the (meth)acrylic monomer component (for example, by increasing the content of the acrylic monomer in the photocurable composition). Furthermore, the storage elastic modulus of the test piece A1 at 37°C can be improved preferentially over the storage elastic modulus of the test piece A1 at 25°C by increasing the content of the methacrylic group among the methacrylic group and acrylic group of the (meth)acrylic monomer component (for example, by increasing the content of the methacrylic monomer in the photocurable composition).

Furthermore, the storage elastic modulus of the test piece A1 at 25°C can be reduced preferentially over the storage elastic modulus of the test piece A1 at 37°C by reducing the content of the acrylic group among the methacrylic group and acrylic group of the (meth)acrylic monomer component (for example, by reducing the content of the acrylic monomer in the photocurable composition). Furthermore, the storage elastic modulus of the test piece A1 at 37°C can be reduced preferentially over the storage elastic modulus of the test piece A1 at 25°C by reducing the content of the methacrylic group among the methacrylic group and acrylic group of the (meth)acrylic monomer component (for example, by reducing the content of the methacrylic monomer in the photocurable composition).

### [Photopolymerization initiator]

The photocurable composition of the present disclosure contains a photopolymerization initiator.

The photocurable composition of the present disclosure may contain only one type of photopolymerization initiator, or may contain two or more types of photopolymerization initiators.

The photopolymerization initiator is not particularly limited as long as it generates radicals when irradiated with light, and the photopolymerization initiator is preferably one which generates radicals when irradiated at a wavelength of light used for photomodeling.

The wavelength of light used for photomodeling is generally, for example, from 365 nm to 500 nm, and from a practical standpoint, it is preferably from 365 nm to 430 nm, and more preferably from 365 nm to 420 nm.

Examples of the photopolymerization initiator include an acylphosphine oxide-based compound, a benzoylformic acid alkyl compound, an alkylphenone-based compound, a titanocene-based compound, an oxime ester-based compound, a benzoin-based compound, an acetophenone-based compound, a benzophenone-based compound, a thioxanthone-based compound, an α-acyloxime ester-based compound, a phenylglyoxylate-based compound, a benzyl-based compound, an azo-based compound, a diphenyl sulfide-based compound, an organic dye-based compound, an iron-phthalocyanine-based compound, a benzoin ether-based compound, an anthraquinone-based compound, and the like.

Examples of the acylphosphine oxide-based compound include 2,4,6-trimethylbenzoyl-diphenyl-phosphine oxide, 2,6-dimethoxybenzoyl-diphenyl-phosphine oxide, bis(2,4,6-trimethylbenzoyl)-phenylphosphine oxide and the like

The total content of the photopolymerization initiator in the photocurable composition of the present disclosure is preferably from 0.1% by mass to 5% by mass, more preferably from 0.5% by mass to 4% by mass, and still more preferably from 0.5% by mass to 3% by mass, with respect to the total amount of the photocurable composition.

In a case in which the photopolymerization initiator contains an acylphosphine oxide-based compound, the content of the acylphosphine oxide-based compound may be from 50% by mass to 100% by mass, may be from 70% by mass to 100% by mass, or may be from 90% by mass to 100% by mass, with respect to the total amount of the photopolymerization initiator.

In a case in which the aforementioned (a) or (b) is satisfied, each of the total content of two or more types of the di(meth)acrylic monomer (A) and the photopolymerization initiator or the total content of the di(meth)acrylic monomer (A), the (meth)acrylic monomer (B) and the photopolymerization initiator is independently preferably 80% by mass or more, more preferably 90% by mass or more, and still more preferably 95% by mass or more, with respect to the total amount of the photocurable composition of the present disclosure.

The upper limit of the total content of two or more types of the di(meth)acrylic monomer (A) and the photopolymerization initiator or the total content of the di(meth)acrylic monomer (A), the (meth)acrylic monomer (B) and the photopolymerization initiator is not particularly limited, and may be 100% by mass or less.

### <Other components>

The photocurable composition of the present disclosure may contain one or more types of other components other than those described above, if necessary.

In a case in which the photocurable composition contains another component, the total mass of the (meth)acrylic monomer component and the photopolymerization initiator is preferably 30% by mass or more, more preferably 50% by mass or more, still more preferably 70% by mass or more, even still more preferably 80% by mass or more, and even still more preferably 90% by mass or more, with respective to the total amount of the photocurable composition.

Examples of the other component include a colorant, a coupling agent such as a silane coupling agent (for example, 3-acryloxypropyltrimethoxysilane), additives such as a rubber agent, an ion trapping agent, an ion exchange agent, a leveling agent, a plasticizer, and a defoaming agent, a thermal polymerization initiator, and the like.

In a case in which the photocurable composition of the present disclosure contains the thermal polymerization initiator, it is possible to combine photocuring and thermal curing for use. Examples of the thermal polymerization initiator include a thermal radical generator, an amine compound, and the like.

Examples of the other component include an inorganic filler.

However, from the viewpoint of further improving the shape accuracy of the cured product, the photocurable composition of the present disclosure preferably contains no inorganic filler (for example, silica, barium borosilicate glass, and the like. The same applies hereinafter.), or in the case of containing an inorganic filler, the content of the inorganic filler with respect to the total amount of the photocurable composition is preferably 60% by mass or less (more preferably 40% by mass or less, still more preferably 20% by mass or less, and particularly preferably 10% by mass or less).

The method of preparation of the photocurable composition of the disclosure is not particularly limited.

Examples of the method for preparing a photocurable composition of the disclosure include a method for mixing a (meth)acrylic monomer component, the photopolymerization initiator and another component if necessary.

The means of mixing each component is not particularly limited, and examples thereof include a means such as dissolution by ultrasonic waves, a twin-arm stirrer, a roll mixer, a twinscrew extruder, a ball mill mixer, or a planetary stirrer.

The photocurable composition of the present embodiment may be prepared by mixing each component, then removing impurities from the mixture with a filter, and further applying vacuum degassing treatment thereto.

### <Preferred viscosity of photocurable composition>

The photocurable composition of the present disclosure preferably has a viscosity of from 5 mPa s to 6,000 mPa·s as measured by an E-type viscometer under the conditions of 25°C and 50 rpm (hereinafter also simply referred to as "viscosity").

Here, rpm refers to revolutions per minute.

In a case of the viscosity being from 5 mPa s to 6,000 mPa·s, the photocurable composition is excellent in handleability thereof upon production of the cured product (in particular the photofabrication product).

The viscosity is more preferably from 10 mPa·s to 5,000 mPa·s, still more preferably from 20 mPa·s to 5,000 mPa·s, and even still more preferably from 100 mPa·s to 4,500 mPa·s.

The photocurable composition of the second embodiment will be described below. Note that the description of the matters common to the aforementioned photocurable composition of the first embodiment will be omitted.

### [Second embodiment]

### [Photocurable composition]

A photocurable composition of the second embodiment of the present disclosure contains a (meth)acrylic monomer component, and a photopolymerization initiator, wherein the (meth)acrylic monomer component comprises:
a mono(meth)acrylic monomer (X) having one (meth)acryloyloxy group and an aromatic ring,
a di(meth)acrylic monomer (Y) having at least one of a cyclic structure or a urethane bond, and two (meth)acryloyloxy groups and not having a siloxane bond, and
a polyfunctional (meth)acrylic monomer (Z) having a siloxane bond and two or more (meth)acryloyloxy groups.

The photocurable composition of the present disclosure contains a (meth)acrylic monomer component and photopolymerization initiator, and the (meth)acrylic monomer component contains a mono(meth)acrylic monomer (X) and a di(meth)acrylic monomer (Y) and a polyfunctional (meth)acrylic monomer (Z). By using such a photocurable composition, it is possible to produce a mold that is suppressed from deforming during the production of a plate denture, or a mold that allows a plate denture to be easily removed from the mold when the plate denture is produced using the mold.

In a case in which a rectangular sheet-like test piece A1 with a length of 40 mm, a width of 10 mm, and a thickness of 1.0 mm is produced by photomodeling under conditions in which the photocurable composition is irradiated with visible light having a wavelength of 405 nm at an irradiation dose of 11 mJ/cm² to form a cured layer A1 with a thickness of 50 µm, the cured layer A1 is stacked in a thickness direction thereof to form a rectangular sheet-like modeling product A1 with a length of 40 mm, a width of 10 mm, and a thickness of 1.0 mm, and the modeling product A1 is irradiated with ultraviolet rays having a wavelength of 365 nm at an irradiation dose of 3 J/cm² to produce the test piece A1, from the viewpoint of suppressing deformation of a mold (for example, from the viewpoint of suppressing deformation of a mold when a photocurable composition for producing denture base is injected into a mold produced by using the photocurable composition of the present disclosure and then polymerized), the storage elastic modulus at 25°C of the test piece A1 may be 10 MPa or more, or from the viewpoint of the removability from a mold (for example, from the viewpoint of suppressing damage to a mold, a plate denture, or the like, when the plate denture is removed from the mold), it may be 100 MPa or less.

From the viewpoint of the removability from a mold, the storage elastic modulus at 37°C of the test piece A1 may be 400 MPa or less, or from the viewpoint of suppressing deformation of a mold when in use, it may be 6 MPa or more.

By using a photocurable composition whose storage elastic modulus at 25°C and storage elastic modulus at 37°C fall within the above numerical ranges, it is possible to more suitably produce a mold in which deformation during the production of a plate denture is suppressed, or a mold from which a plate denture is easily removed when the plate denture is producing by using the mold.

In the photocurable composition of the second embodiment, the storage elastic modulus at 25°C of the test piece A1 may be 10 MPa or less, for example, may be from 1 MPa to 10 MPa, or may be from 2 MPa to 8 MPa.

In the photocurable composition of the second embodiment, the storage elastic modulus at 37°C of the test piece A1, for example, may be from 0.5 MPa to 20 MPa, may be from 1 MPa to 10 MPa, or may be from 1 MPa to 6 MPa.

In a case in which the cured product of the photocurable composition of the second embodiment is used as a mold, the releasability of a member (e.g., a plate denture), which is produced using the mold, from the mold is improved, and the toughness when demolding is improved, making the cured product less likely to break.

The (meth)acrylic monomer component contains a mono(meth)acrylic monomer (X) having one (meth)acryloyloxy group and an aromatic ring. There is a tendency that a plate denture is easily removed from a mold (the releasability is improved), by using the mono(meth)acrylic monomer (X) and thus improving an aromatic ring concentration in the (meth)acrylic monomer component, and there is a tendency that breakage when a plate denture is removed from a mold can be suppressed (the toughness is improved). Furthermore, a mold in which deformation during the production of a plate denture is suppressed can be suitably produced, and the flexibility of the mold can be increased by using the monofunctional monomer, so that the shape recovery of the mold can also be improved. Furthermore, the mono(meth)acrylic monomer (X) is suitably used when adjusting the storage elastic modulus at 25°C or the storage elastic modulus at 37°C of the cured product of the photocurable composition to a low value.

The molecular weight of the mono(meth)acrylic monomer (X) may be from 160 to 400, or may be from 180 to 300.

The average molecular weight of the mono(meth)acrylic monomer (X) may be from 160 to 400, or may be from 180 to 300.

Two types of mono(meth)acrylic monomers (X) having different molecular weights may be used in combination, which tends to increase the dispersibility of the mono(meth)acrylic monomer (X) in the polymer and improve the shape recovery speed.

The mono(meth)acrylic monomer (X) is not particularly limited as long as it is a compound having one (meth)acryloyloxy group and an aromatic ring, and for example, may be a compound represented by the following Formula (5).

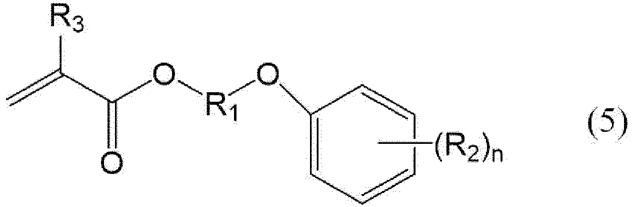

In Formula (5), R₁ is a divalent linking group, R₂ is an alkyl group or an aryl group which may have a substituent, R₃ is a hydrogen atom or a methyl group, and n is an integer of from 0 to 5.

In formula (5), R₁ may be an alkylene group, an arylene group (such as a phenylene group), an alkylenearylene group (such as an alkylenephenylene group), an arylenealkylene group (such as a phenylenealkylene group), an alkyleneoxy group, an aryleneoxy group, or a combination of two or more of these. Furthermore, the hydrogen atoms contained in R₁ may be substituted with a hydroxy group, an alkyl group, an aryl group, an amino group, or the like.

In Formula (5), the number of atoms in the main chain of R₁ may be from 1 to 20, or may be from 2 to 10. The number of carbon atoms in R₁ may be from 1 to 20, or may be from 2 to 10.

In Formula (5), n is preferably 0 or 1. In a case in which n is from 1 to 5, R₂ is preferably a phenyl group which may have a substituent. The number of carbon atoms in R₂ may be from 1 to 20, or may be from 1 to 10. Examples of the substituent, which the alkyl group or aryl group in R₂ may have, include a hydroxy group, an alkyl group, an aryl group, and an amino group.

The (meth)acrylic monomer component contains a di(meth)acrylic monomer (Y) having at least one of a cyclic structure or a urethane bond, and two (meth)acryloyloxy groups and not having a siloxane bond. The di(meth)acrylic monomer (Y) having a cyclic structure preferably contributes to suppression of deformation of a mold during the production of a plate denture and to improvement of the water resistance of the mold. In addition, in a case in which the cyclic structure contains an aromatic ring, the aromatic ring concentration in the (meth)acrylic monomer component increases, and the mold releasability increases and the toughness tends to be excellent. The di(meth)acrylic monomer (Y) having a urethane bond can suppress breakage when a plate denture is removed from a mold, and tends to be excellent in toughness. In addition, the di(meth)acrylic monomer (Y) is preferably used when adjusting the storage elastic modulus at 25°C or storage elastic modulus at 37°C of the cured product of the photocurable composition to a high value.

The di(meth)acrylic monomer (Y) is not particularly limited as long as it has at least one of a cyclic structure or a urethane bond, two (meth)acryloyloxy groups, and no siloxane bond. Examples of the cyclic structure include an aromatic cyclic structure and an alicyclic structure.

The di(meth)acrylic monomer (Y) may have a distance of from 10 Å to 200 Å between an oxygen atom forming an oxy group in one of the (meth)acryloyloxy groups and an oxygen atom forming an oxy group in another of the (meth)acryloyloxy groups. The aforementioned distance between the oxygen atoms may be 10 Å or more and less than 25 Å, may be from 25 Å to 80 Å, or may be from more than 80 Å and less than 200 Å. For example, there is a tendency that deformation of a mold during the production of a plate denture can be suitably suppressed by making the aforementioned distance between the oxygen atom lower, and thus improving an elastic modules of the mold, and there is a tendency that the releasability can be improved by making the aforementioned distance between the oxygen atom longer.

The molecular weight of the di(meth)acrylic monomer (Y) may be from 400 to 5000.

The weight average molecular weight of the di(meth)acrylic monomer (Y) may be from 400 to 5000.

Two types of di(meth)acrylic monomers (Y) having different molecular weights may be used in combination, which tends to increase the dispersibility of the di(meth)acrylic monomer (Y) in the polymer and improve the shape recovery speed.

In a case in which the di(meth)acrylic monomer (Y) contains a urethane bond, it may be a compound represented by the following Formula (6-1), or in a case in which the di(meth)acrylic monomer (Y) contains a cyclic structure, it may be a compound represented by the following Formula (6-2). In a case in which the di(meth)acrylic monomer (Y) contains both a urethane bond and a cyclic structure, it may be a compound represented by the following Formula (6-1).

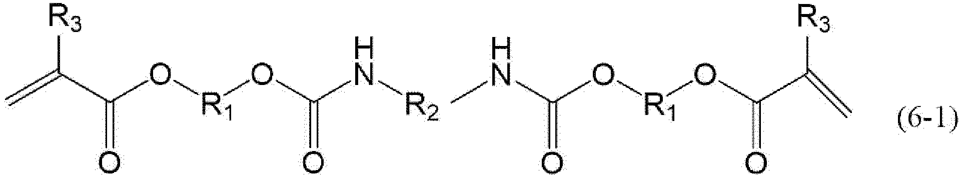

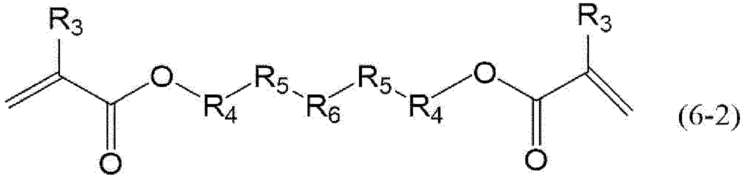

In Formula (6-1), R₁'s each independently represent an alkylene group, an ester bond, an alkyleneoxy group, or a combination of at least two or more of these, each of which may have a substituent; R₂'s each independently represent an alkylene group, a divalent cyclic structure, an ester bond, a urethane bond, an alkyleneoxy group, or a combination of at least two or more of these, each of which may have a substituent; and R₃'s each independently represent a hydrogen atom or a methyl group.

In Formula (6-2), R₃'s are each independently a hydrogen atom or a methyl group, R₄'s are each independently an alkylene group, an alkyleneoxy group, or a combination thereof, each of which may have a substituent, R₅'s are each independently an oxygen atom or an ester bond (*1-O-C(=O)-*2, * 1 is the bonding position to R₄, and *2 is the bonding position to R₆), and R₆ is a divalent linking group containing a cyclic structure.

In Formula (6-1), in a case in which R₁ is an alkylene group which may have a substituent or contains such an alkylene group, examples of the substituent include a phenyloxy group.

In a case in which R₁ is an alkyleneoxy group or contains such an alkyleneoxy group, the alkyleneoxy group may be an ethyleneoxy group, a propyleneoxy group, or the like. In a case in which R₁ contains a plurality of alkyleneoxy groups, the plurality of alkyleneoxy groups may be polyethyleneoxy groups, polypropyleneoxy groups, or the like.

In a case in which R₁ has an ester bond, R₁ may contain a structural unit derived from ε-caprolactone, or may contain a plurality of structural units derived from ε-caprolactone. In a case in which R₁ has an ester bond, it may be an alkylene group-O-CO-alkylene group, of which the -O-CO-alkylene group may have a repeating structure (for example, from 2 to 10).

The number of carbon atoms in R₁ may be from 1 to 50, or may be from 2 to 25.

In Formula (6-1), in a case in which R₂ is an alkylene group (which may be linear or branched) which may have a substituent, examples of the substituent include a hydroxy group, an alkyl group, an aryl group, an amino group, and the like.

In Formula (6-1), in case in which R₂ is a divalent cyclic structure or contains a divalent cyclic structure, examples of the cyclic structure include an aromatic ring or an alicyclic ring, and specific examples include a phenylene group and a cyclohexylene group. R₂ may also contain a group formed by a divalent cyclic structure and a divalent alkylene group (for example, an isophorone group, a methylenebis(cyclohexylene) group).

R₂ may also be a divalent hydrocarbon group containing two cyclic structures, or a divalent linking group containing two urethane bonds and an alkyleneoxy group (for example, a divalent hydrocarbon group containing a cyclic structure-urethane bond-(poly)alkyleneoxy group-urethane bond-divalent hydrocarbon group containing a cyclic structure).

The number of carbon atoms in R₂ may be from 1 to 200, or may be from 2 to 100.

In Formula (6-2), R₄ may be an alkyleneoxy group and R₅ may be an oxygen atom, or R₄ may be an alkylene group and R₅ may be an ester bond (*1-O-C(=O)-*2, *1 is the bonding position with R₄ and *2 is the bonding position with R₆). R₆ may be a phenylene group or a bisphenol skeleton (e.g., a bisphenol A skeleton or a bisphenol F skeleton).

The number of carbon atoms in R₄ may be from 1 to 50, or may be from 2 to 30. The number of carbon atoms in R₆ may be from 1 to 50, or may be from 2 to 20.

The (meth)acrylic monomer component contains a polyfunctional (meth)acrylic monomer (Z) having a siloxane bond (Si-O-Si) and two or more (meth)acryloyloxy groups. There is a tendency that a plate denture is easily removed from a mold (the releasability is improved), by containing the polyfunctional (meth)acrylic monomer (Z) and thus improving an aromatic ring concentration in the (meth)acrylic monomer component, and there is a tendency that, when producing a plate denture, deformability is excellent and dimensional accuracy is excellent. In addition, the polyfunctional (meth)acrylic monomer (Z) is preferably used when adjusting the storage elastic modulus at 25°C or storage elastic modulus at 37°C of the cured product of the photocurable composition to a low value.

The polyfunctional (meth)acrylic monomer (Z) has a siloxane bond (Si-O-Si) and two or more (meth)acryloyloxy groups. The polyfunctional (meth)acrylic monomer (Z) may contain a plurality of siloxane bonds (Si-O-Si), more specifically, may contain linear siloxane bonds, ladder-shaped siloxane bonds including linear and branched structure, cage-shaped siloxane bonds, or the like. The polyfunctional (meth)acrylic monomer (Z) may contain two or three or more (meth)acryloyloxy groups.

Examples of the siloxane bond include a dimethylsiloxane bond, a methylphenylsiloxane bond, and a diphenylsiloxane bond, the dimethylsiloxane bond is preferable.

The polyfunctional (meth)acrylic monomer (Z) may be a compound containing a siloxane bond (Si-O-Si), and three (meth)acryloyloxy groups, or may be a silsesquioxane containing three or more (meth)acryloyloxy groups.

The molecular weight of the polyfunctional (meth)acrylic monomer (Z) may be from 400 to 5000.

The weight average molecular weight of the polyfunctional (meth)acrylic monomer (Z) may be from 400 to 4000.

The polyfunctional (meth)acrylic monomer (Z) may be a compound represented by the following Formula (7).

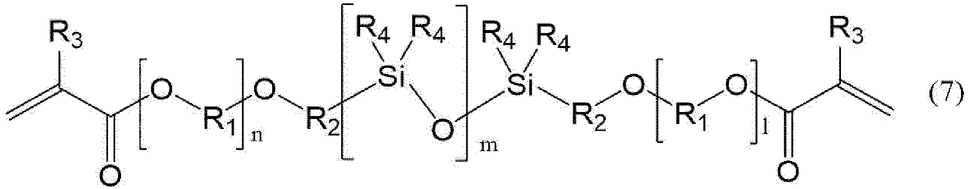

In Formula (7), each R₁ is independently an alkylene group which may have a substituent, each R₂ is independently an alkylene group which may have a substituent, each R₃ is independently a hydrogen atom or a methyl group, each R₄ is independently an alkyl group, a hydrogen atom or an aryl group, m is an integer of 0 or more, n is an integer of 0 or more, and 1 is an integer of 0 or more.

R₁ is preferably a methylene group, an ethylene group, or a propylene group, and R₂ is more preferably a methylene group, an ethylene group, a propylene group, or a butylene group.
R₄ is preferably a methyl group or a phenyl group, and more preferably a methyl group.
m may be from 1 to 30, or may be from 2 to 20. From the viewpoint of compatibility with another (meth)acrylic monomer component, m is preferably 30 or less, and more preferably 20 or less.
n may be 0 or 1 or more. In a case in which n is 1 or more, n may be from 1 to 30, or may be from 1 to 20.
l may be 0 or 1 or more. In a case in which l is 1 or more, l may be from 1 to 30, or may be from 1 to 20.

In the (meth)acrylic monomer component, the content of the mono(meth)acrylic monomer (X) is preferably from 30% by mass to 90% by mass, and more preferably from 40% by mass to 80% by mass, with respect to the total amount of the (meth)acrylic monomer component.

In the (meth)acrylic monomer component, the content of the di(meth)acrylic monomer (Y) is preferably from 5% by mass to 55% by mass, and more preferably from 10% by mass to 40% by mass, with respect to the total amount of the (meth)acrylic monomer component.

In the (meth)acrylic monomer component, the content of the polyfunctional (meth)acrylic monomer (Z) is preferably from 1% by mass to 60% by mass, and more preferably from 5% by mass to 50% by mass, with respect to the total amount of the (meth)acrylic monomer component.

In the (meth)acrylic monomer component, the total content of the mono(meth)acrylic monomer (X), the di(meth)acrylic monomer (Y) and the polyfunctional (meth)acrylic monomer (Z) is preferably from 50% by mass to 100% by mass, more preferably from 70% by mass to 100% by mass, and still more preferably from 90% by mass to 100% by mass.

The siloxane bond concentration in the composition is preferably from 0.100 mmol/g to 3.000 mmol/g, and 0.300 mmol/g to 2.500 mmol/g.

In a case in which the siloxane bond concentration is 0.100 mmol/g or more, releasability tends to be improved, and in a case in which the siloxane bond concentration is 3.000 mmol/g or less, breakage tends to be suppressed when removing a plate denture from a mold and the mold tends to be excellent in toughness.

The aromatic ring concentration in the (meth)acrylic monomer component is preferably from 0.0015 mmol/g to 0.0070 mmol/g, and more preferably from 0.0020 mmol/g to 0.0065 mmol/g.

From the viewpoint of releasability and toughness, it is preferred that the siloxane bond concentration in the composition and the aromatic ring concentration in the (meth)acrylic monomer component each satisfy the aforementioned numerical ranges.

The total content of the mono(meth)acrylic monomer (X), the di(meth)acrylic monomer (Y) the polyfunctional (meth)acrylic monomer (Z) and the photopolymerization initiator is preferably 80% by mass or more, more preferably 90% by mass or more, and still more preferably 95% by mass or more, with respect to the total amount of the photocurable composition of the present disclosure.

The upper limit of the total content of the mono(meth)acrylic monomer (X), the di(meth)acrylic monomer (Y) the polyfunctional (meth)acrylic monomer (Z) and the photopolymerization initiator is not particularly limited and may be 100% by mass or less.

### [Three-dimensional modeling product]

A three-dimensional modeling product of the present disclosure includes a cured product of the photocurable composition of the present disclosure.

Therefore, in a case in which the three-dimensional modeling product of the present disclosure is a mold, deformation during the production of a plate denture can be suppressed, when a plate denture is produced using a mold, the plate denture can be easily removed from the mold.

The three-dimensional modeling product of the present disclosure preferably includes a cured product by photomodeling (i.e., a photofabrication product).

A method of producing a cured product (e.g., a photofabrication product) is as described above.

A preferred embodiment of the three-dimensional modeling product is a mold, and more specifically, a mold used in the production of a plate denture.

### [Method of producing cured product]

A method of producing a cured product of the present disclosure includes a step of polymerizing a curable composition in the aforementioned mold. For example, a mold is produced using the aforementioned photocurable composition of the present disclosure, a curable composition is injected into the produced mold and then a cured product is produced by polymerizing the injected curable composition. The curable composition injected into the mold is not particularly limited as long as it contains a polymerizable component that is polymerized by heat, light, or the like. For example, in a case in which artificial teeth are arranged into the mold to produce a plate denture, a conventionally known curable composition for producing a denture base may be injected into the mold, and the curable composition for producing a denture base after injection may be cured.

### [Method of producing plate denture]

A method of producing a plate denture of the present disclosure includes a step of curing a photocurable composition by photomodeling to produce a mold used in a production of a plate denture, and a step of polymerizing a curable composition in the mold to produce a plate denture.

The method of producing a plate denture of the present disclosure is a method of producing a plate denture by going through a method of producing a mold a method of producing the plate denture, and it is possible to produce a plate denture with a simple method compared with a conventional method of producing a plate denture using a mold.

It is preferable that the step of producing a mold includes, for example, a step of obtaining three-dimensional impression data of the oral cavity of a plate denture user, a step of obtaining mold data from the obtained three-dimensional impression data, and a step of curing a photocurable composition by photomodeling based on the obtained mold data.

It is preferable that the step of producing a plate denture includes a step of arranging artificial teeth in a mold, a step of injecting a curable composition for producing a denture base into the mold with the artificial teeth arranged in the mold, a step of curing the curable composition after injection, and a step of removing the produced plate denture from the mold.

In a method of producing a plate denture of the present disclosure, a photocurable composition used for the production of a mold is not particularly limited. For example, from the viewpoint of suppressing deformation of a mold in the step of curing the curable composition after injection, and from the viewpoint of suppressing damage to a mold, a plate denture, or the like in the step of removing the produced plate denture from the mold, the aforementioned photocurable composition of the present disclosure is preferably used as the photocurable composition used in producing a mold.

### EXAMPLES

Examples of the disclosure will now be described; however, the disclosure is not limited to the below-described Examples.

### [Examples 1-19, Comparative Examples 1-3]

### <Preparation of photocurable compositions>

The components shown in Tables 1 to 3 were mixed to obtain photocurable compositions. Table 1 shows the details of each component, and Tables 2 and 3 show the mixing ratios of each component.

### <Measurements and evaluations>

The following measurements and evaluations were performed using the obtained photocurable compositions.

The results are shown in Tables 2 and 3.

### (Storage elastic modulus)

A rectangular sheet-like test piece A1 with a length of 40 mm, a width of 10 mm, and a thickness of 1.0 mm was produced by photomodeling under conditions in which each of the photocurable compositions was irradiated with visible light having a wavelength of 405 nm at an irradiation dose of 11 mJ/cm² to form a cured layer A1 with a thickness of 50 µm, the cured layer A1 was stacked in a thickness direction thereof to form a rectangular sheet-like modeling product A1 with a length of 40 mm, a width of 10 mm, and a thickness of 1.0 mm, and the modeling product A1 was irradiated with ultraviolet rays having a wavelength of 365 nm at an irradiation dose of 3 J/cm² to produce the test piece A1.

The produced test piece A1 was subjected to dynamic viscoelasticity measurement at a temperature rise range of 25°C to 200°C and a temperature rise rate of 3°C/min at a measurement frequency of 1 Hz to determine the storage elastic modulus at 25°C and the storage elastic modulus at 37°C.

The test piece A1 was produced using a DLP 3D printer (Kulzer GmbH, Cara Print 4.0), and the storage elastic modulus was measured using a dynamic viscoelasticity measuring device (Hitachi High-Tech Science Corporation, DMA7100).

### (Viscosity)

The viscosity of the obtained photocurable compositions was measured using an E-type viscometer under conditions of 25°C and 50 rpm.

As a result, the viscosities of the photocurable compositions of Examples 1 to 19 were all in the range of 50 mPa·s to 3,000 mPa·s.

### (Deformability)

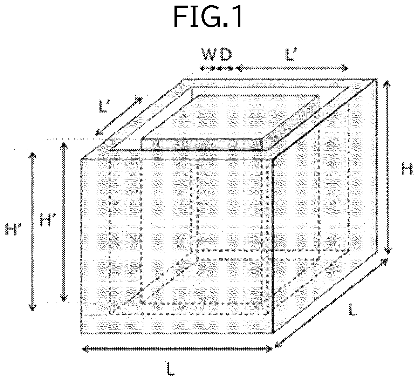

A test piece A2 shown in FIG. 1 was produced by photomodeling under conditions in which each of the obtained photocurable composition was irradiated with visible light having a wavelength of 405 nm at an irradiation dose of 11 mJ/cm² to form a cured layer A1 with a thickness of 50 µm, the cured layer A1 was stacked in a thickness direction thereof to form a three-dimensional modeling product A2 shown in FIG. 1, and the modeling product A2 was irradiated with ultraviolet rays having a wavelength of 365 nm at an irradiation dose of 3 J/cm² to produce the test piece A2.

As shown in FIG. 1, the test piece A2 has a shape in which L is 28 mm, L' is 20 mm, W is 2 mm, H is 24 mm, H' is 22 mm, and the width (D) is 2 mm. A (meth)acrylate polymer powder for plate denture production and Palapress (registered trademark) Vario (manufactured by Kulzer GmbH), which was (meth)acrylate monomer liquid, were mixed in a ratio of 10 g powder: 7 mL liquid, and after the expansion stage (about 2 minutes at 23°C), the mixture was filled into the 2 mm wide gap of the produced test piece A2, and polymerized at 55°C and 2 bar pressure for 30 minutes. The length L of the test piece A2 was then measured with a micrometer (MDC-25PX manufactured by Mitutoyo Corporation), and the deviation (mm) from the design value (28 mm) was calculated. The smaller the deviation from the design value, the better the deformability during the production of a plate denture and dimensional accuracy. Deviation from the design value was evaluated as "A" if it was less than 0.05 mm, "B" if it was 0.05 mm or more and 0.10 mm or less, and "C" if it was more than 0.10 mm.

### (Shape recovery in removal test at 37°C)

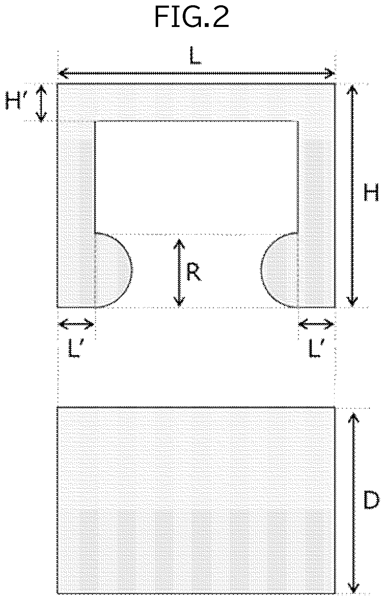

A test piece A3 shown in FIG. 2 was produced by photomodeling under conditions in which each of the obtained photocurable composition was irradiated with visible light having a wavelength of 405 nm at an irradiation dose of 11 mJ/cm² to form a cured layer A1 with a thickness of 50 µm, the cured layer A1 was stacked in a thickness direction thereof to form a three-dimensional modeling product A3 shown in FIG.2, and the modeling product A3 was irradiated with ultraviolet rays having a wavelength of 365 nm at an irradiation dose of 3 J/cm² to produce the test piece A3.

As shown in FIG. 2, the test piece A3 has a shape in which L is 15 mm, L' is 2 mm, H is 12 mm, H' is 2 mm, R is 4 mm and the thickness (D) is 10 mm.

An iron ball (diameter 10 mm) was inserted and removed between the two semi-cylinders of the obtained test piece A3 at a moving speed of 120.0±2.0 mm/min to perform a mounting/removal test. After 10 insertion and removal movements, the test piece was observed and evaluated as "A" if there was no change in shape and no cracks after the test, "B" if there was a change in shape and no cracks after the test, and "C" if a crack occurred after the test.

**[Table 1]**

| | Component | | Name | Mw | O-O interatomic distance d1(Å) | Number of aromatic ring in monomer | Number of urethane bond in monomer | Number of hydroxy group in monomer |
|---|---|---|---|---|---|---|---|---|
| Composition | Di(meth)acrylic monomer (A) | | A-BPE-10 | 776.92 | 33.7 | 2 | 0 | 0 |
| | | | BPE-500 | 804.97 | 33.7 | 2 | 0 | 0 |
| | (Meth)acrylic monomer (B) | Monomer(B-1) | BP-4EAL | 512.6 | 19.1 | 2 | 0 | 0 |
| | | | SR540 | 540.65 | 19.1 | 2 | 0 | 0 |
| | | | M-208 | 512.6 | 20.6 | 2 | 0 | 0 |
| | | | SR348 | 452.55 | 13.5 | 2 | 0 | 0 |
| | | | UDA | 442.51 | 14.8 | 0 | 2 | 0 |
| | | | UDMA | 470.56 | 14.8 | 0 | 2 | 0 |
| | | | AH-600 | 612.68 | 14.4 | 2 | 2 | 0 |
| | | Monomer(B-2) | UA1 | 1632.21 | 96.3 | 0 | 4 | 0 |
| | | Monomer(B-3) | PO-A | 192.21 | - | 1 | 0 | 0 |
| | | | PO | 206.24 | - | 1 | 0 | 0 |
| | | | POB-A | 254.29 | - | 2 | 0 | 0 |
| | | | A-LEN-10 | 268.31 | - | 2 | 0 | 0 |
| | | | 4-HBA | 144.17 | - | 0 | 0 | 1 |
| | | | HOP-A | 130.14 | - | 0 | 0 | 1 |
| | | | M-600A | 222.24 | - | 1 | 0 | 1 |
| | Other polymer | | HBPEM-10 | 817.07 | 43.8 | 0 | 0 | 0 |
| | | | HBPE-4 | 552.75 | 23.4 | 0 | 0 | 0 |
| | Photopolymerization initiator | | Omnirad819 | 348.38 | - | - | - | - |
| | | | OmniradTPO | 418.47 | - | - | - | - |

**[Table 2]**

| | Component | | Name | Comparative Example | | | Example | | | | | | | |
|---|---|---|---|---|---|---|---|---|---|---|---|---|---|---|
| | | | | 1 | 2 | 3 | 1 | 2 | 3 | 4 | 5 | 6 | 7 | 8 |
| Composition | Di(meth)acrylic monomer (A) | | A-BPE-10 | | | | 70 | 50 | 60 | 60 | 60 | 80 | 80 | 70 |
| | | | BPE-500 | | | | 30 | 50 | | | | | | |
| | (Meth)acrylic monomer (B) | Monomer (B-1) | BP-4EAL | | | | | | 40 | | | | | |
| | | | SR540 | | 50 | | | | | 40 | | | | |
| | | | M-208 | | | | | | | | 40 | | | |
| | | | SR348 | | 50 | | | | | | | | | |
| | | | UDA | | | | | | | | | 20 | | |
| | | | UDMA | | | | | | | | | | 20 | |
| | | | AH-600 | | | | | | | | | | | 30 |
| | | Monomer (B-2) | UA1 | | | 40 | | | | | | | | |
| | | Monomer (B-3) | PO-A | | | | | | | | | | | |
| | | | PO | | | | | | | | | | | |
| | | | POB-A | | | 60 | | | | | | | | |
| | | | A-LEN-10 | | | | | | | | | | | |
| | | | 4-HBA | | | | | | | | | | | |
| | | | HOP-A | | | | | | | | | | | |
| | | | M-600A | | | | | | | | | | | |
| | Other polymer | | HBPEM-10 | 70 | | | | | | | | | | |
| | | | HBPE-4 | 30 | | | | | | | | | | |
| | Photopolymerization initiator | | Omnirad819 | 1 | 2 | | 1 | 1 | 1 | 1 | 1 | 1 | 1 | 1 |
| | | | OmniradTPO | | | 2 | | | | | | | | |
| Total | | | | 101 | 102 | 102 | 101 | 101 | 101 | 101 | 101 | 101 | 101 | 101 |
| Storage elastic modulus at 25°C (MPa) | | | | 725 | 2572 | 6 | 84 | 102 | 550 | 720 | 451 | 106 | 142 | 468 |
| Storage elastic modulus at 37°C (MPa) | | | | 415 | 2322 | 4 | 59 | 85 | 180 | 216 | 145 | 57 | 59 | 150 |
| Aromatic ring concentration in all monomers (mol/g) | | | | 0.0000 | 0.0040 | 0.0046 | 0.0025 | 0.0025 | 0.0031 | 0.0030 | 0.0031 | 0.0020 | 0.0020 | 0.0028 |
| Urethane bond concentration in all monomers (mol/g) | | | | 0.0000 | 0.0000 | 0.0010 | 0.0000 | 0.0000 | 0.0000 | 0.0000 | 0.0000 | 0.0009 | 0.0008 | 0.0010 |
| Hydroxy group concentration in all monomers (mol/g) | | | | 0.0000 | 0.0000 | 0.0000 | 0.0000 | 0.0000 | 0.0000 | 0.0000 | 0.0000 | 0.0000 | 0.0000 | 0.0000 |
| Deformability A : <0.05mm, B : 0.05mm-0.10mm, C : >0.10mm | | | | B | B | C | A | A | A | A | A | A | A | A |
| Shape recovery in removal test at 37° C A: no change in shape and no cracks, B : change in shape, C : crack | | | | C | C | B | A | A | A | B | A | A | A | A |

**[Table 3]**

| | Component | | Name | Example | | | | | | | | | | |
|---|---|---|---|---|---|---|---|---|---|---|---|---|---|---|
| | | | | 9 | 10 | 11 | 12 | 13 | 14 | 15 | 16 | 17 | 18 | 19 |
| Compositio n | Di(meth)acrylic monomer (A) | | A-BPE-10 | 80 | | 80 | 60 | 70 | 80 | 80 | 50 | | 70 | |
| | | | BPE-500 | | 60 | | | | | | | 60 | | 70 |
| | (Meth)acryli c monomer (B) | Monomer (B-1) | BP-4EAL | | | | | | | | | | | |
| | | | SR540 | | | | | | | | 20 | | | |
| | | | M-208 | | | | | | | | | | | |
| | | | SR348 | | | | | | | | | | | |
| | | | UDA | | | | | | | | | | | |
| | | | UDMA | | | | 20 | | | | | | 10 | 30 |
| | | | AH-600 | | | | | | | | | | | |
| | | Monomer (B-2) | UA1 | 20 | | | | | | | | | | |
| | | Monomer (B-3) | PO-A | | 40 | | | | | | | | | |
| | | | PO | | | 20 | | | | | 30 | 20 | 20 | |
| | | | POB-A | | | | | | | | | | | |
| | | | A-LEN-10 | | | | 20 | | | | | 20 | | |
| | | | 4-HBA | | | | | 30 | | | | | | |
| | | | HOP-A | | | | | | 20 | | | | | |
| | | | M-600A | | | | | | | 20 | | | | |
| | Other polymer | | HBPEM-10 | | | | | | | | | | | |
| | | | HBPE-4 | | | | | | | | | | | |
| | Photopolymerization initiator | Omnirad819 | 1 | 1 | 1 | 1 | 1 | 1 | 1 | 1 | 1 | 1 | 1 | |
| | | OmniradTPO | | | | | | | | | | | | |
| Total | | | 101 | 101 | 101 | 101 | 101 | 101 | 101 | 101 | 101 | 101 | 101 | |
| Storage elastic modulus at 25°C (MPa) | | | 19 | 349 | 301 | 88 | 39 | 87 | 128 | 548 | 213 | 146 | 862 | |
| Storage elastic modulus at 37°C (MPa) | | | 15 | 116 | 167 | 58 | 34 | 59 | 97 | 166 | 106 | 81 | 362 | |
| Aromatic ring concentration in all monomers (mol/g) | | | 0.0020 | 0.0035 | 0.0030 | 0.0030 | 0.0018 | 0.0020 | 0.0029 | 0.0034 | 0.0039 | 0.0027 | 0.0017 | |
| Urethane bond concentration in all monomers (mol/g) | | | 0.0005 | 0.0000 | 0.0000 | 0.0008 | 0.0000 | 0.0000 | 0.0000 | 0.0000 | 0.0000 | 0.0004 | 0.0013 | |
| Hydroxy group concentration in all monomers (mol/g) | | | 0.0000 | 0.0000 | 0.0000 | 0.0000 | 0.0021 | 0.0015 | 0.0009 | 0.0000 | 0.0000 | 0.0000 | 0.0000 | |
| Deformability A : <0.05mm, B : 0.05mm-0.10mm, C : >0.10mm | | | B | A | A | A | A | A | A | A | B | A | A | |
| Shape recovery in removal test at 37°C A: no change in shape and no cracks, B : change in shape, C : crack | | | A | A | A | A | A | A | A | A | A | A | B | |

In Tables 2 and 3, the numbers in the "Composition" column for each Example and Comparative Example mean parts by mass, and a blank space means that the corresponding component is not contained.

### <Di(meth)acrylic monomer (A)>

In Tables 1 to 3, compounds classified as the di(meth)acrylic monomer (A) are specifically the photopolymerizable components 1 and 2 described below.
Photopolymerizable component 1 : Ethoxylated bisphenol A diacrylate (A-BPE-10, Shin-Nakamura Chemical Co., Ltd.)
Photopolymerizable component 2 : Ethoxylated bisphenol A dimethacrylate (BPE-500, Shin-Nakamura Chemical Co., Ltd.)

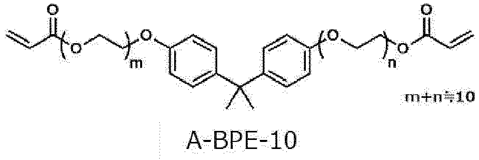

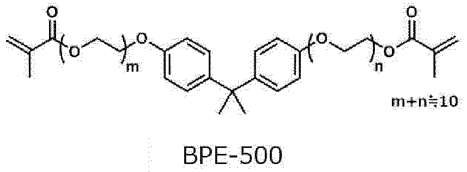

### <(Meth)acrylic monomer (B)>

In Tables 1 to 3, compounds classified as the (meth)acrylic monomer (B) are specifically the photopolymerizable components 3 to 17 described below.

Each of the photopolymerizable components 3 to 9 is classified as the di(meth)acrylic monomer (B-1) having two (meth)acryloyloxy groups, and at least one of an aromatic ring or a urethane bond, having the distance d1 of 10 Å or more and less than 25 Å.

The photopolymerizable component 10 is classified as the di(meth)acrylic monomer (B-2) having two (meth)acryloyloxy groups, and at least one of an aromatic ring or a urethane bond, having the distance d1 of more than 80 Å and less than 200 Å.

Each of the photopolymerizable components 11 to 17 is classified as the mono(meth)acrylic monomer (B-3) having one (meth)acryloyloxy group, and at least one of an aromatic ring or a hydroxy group.
Photopolymerizable component 3 : Ethoxylated bisphenol A diacrylate (BP-4EAL, kyoeisha Chemical Co., Ltd.)
Photopolymerizable component 4 : Ethoxylated bisphenol A dimethacrylate (SR-540, Sartomer Corporation)
Photopolymerizable component 5 : Ethoxylated bisphenol F diacrylate (M-208, Toagosei Company, Limited)
Photopolymerizable component 6 : Ethoxylated bisphenol A dimethacrylate (SR-348, Sartomer Corporation)
Photopolymerizable component 7 : Urethane diacrylate (SUA-1 (UDA in Table), compound produced according to Producing Example 1A described below)
Photopolymerizable component 8 : Urethane dimethacrylate (SUA-2 (UDMA in Table), compound produced according to Producing Example 1B described below)
Photopolymerizable component 9 : Bifunctional urethane acrylate (AH-600, kyoeisha Chemical Co., Ltd.)
Photopolymerizable component 10 : Polyfunctional urethane acrylate (SUA-3 (UA1 in Table), compound produced according to Producing Example 1C described below)
Photopolymerizable component 11 : Phenoxyethyl acrylate (PO-A, kyoeisha Chemical Co., Ltd.)
Photopolymerizable component 12 : Phenoxyethyl methacrylate (PO, kyoeisha Chemical Co., Ltd.)
Photopolymerizable component 13 : m-Phenoxybenzyl acrylate (POB-A, kyoeisha Chemical Co., Ltd.)
Photopolymerizable component 14 : Ethoxylated o-phenylphenol acrylate (A-LEN-10, Shin-Nakamura Chemical Co., Ltd.)
Photopolymerizable component 15 : 4-Hydroxy butyl acrylate (4-HBA)
Photopolymerizable component 16 : 2-Hydroxy propyl acrylate (HOP-A, kyoeisha Chemical Co., Ltd.)
Photopolymerizable component 17 : 2-Hydroxy-3-phenoxypropyl acrylate (M600-A, kyoeisha Chemical Co., Ltd.)

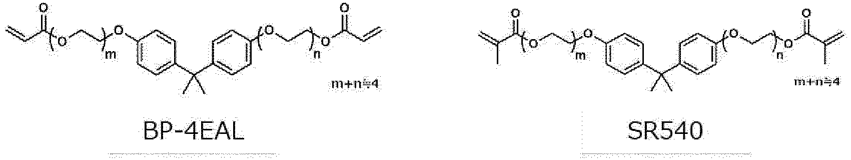

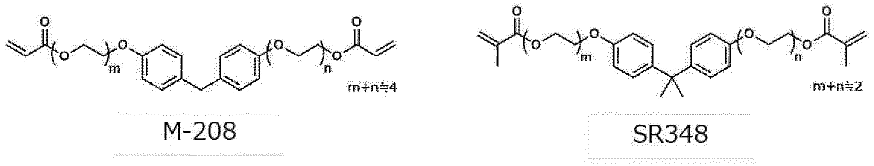

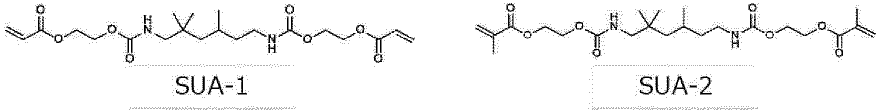

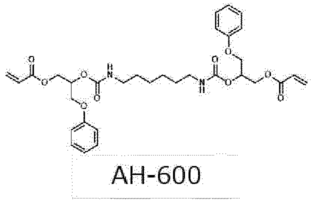

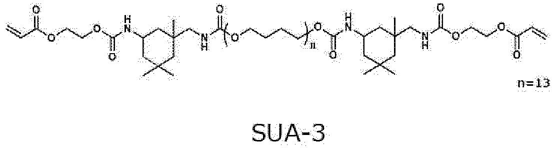

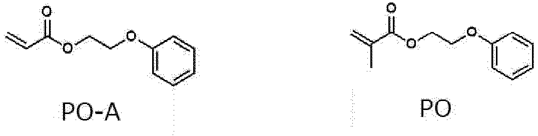

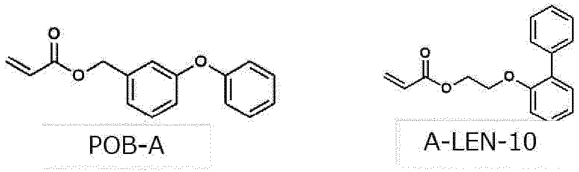

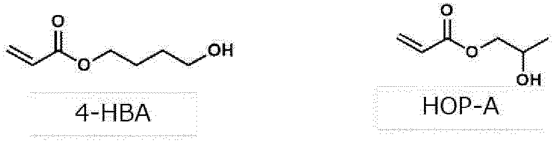

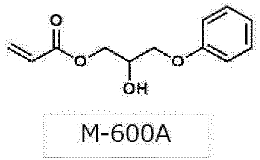

### <Other monomers>

In Tables 1 to 3, compounds classified as other monomers are specifically photopolymerizable components 18 and 19 described below.
Photopolymerizable component 18 : Ethoxylated hydrogenated bisphenol A dimethacrylate (HBPEM-10, DKS Co. Ltd.)
Photopolymerizable component 19: Ethoxylated hydrogenated bisphenol A diacrylate (HBPE-4, DKS Co. Ltd.)

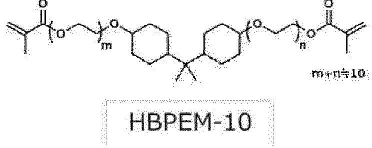

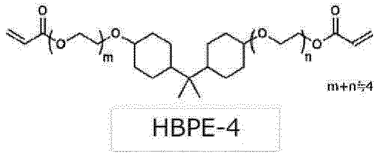

### <Photopolymerization initiator>

In Tables 1 to 3, compounds classified as a photopolymerization initiator is specifically photopolymerization initiators 1 and 2 described below.
Photopolymerization initiator 1 : Acylphosphine oxide compound (OMNIRAD 819: "OMNIRAD 819" manufactured by IGM Resins B.V.)
Photopolymerization initiator 2 : Acylphosphine oxide compound (OMNIRAD TPO: "OMNIRAD TPO" manufactured by IGM Resins B.V.)

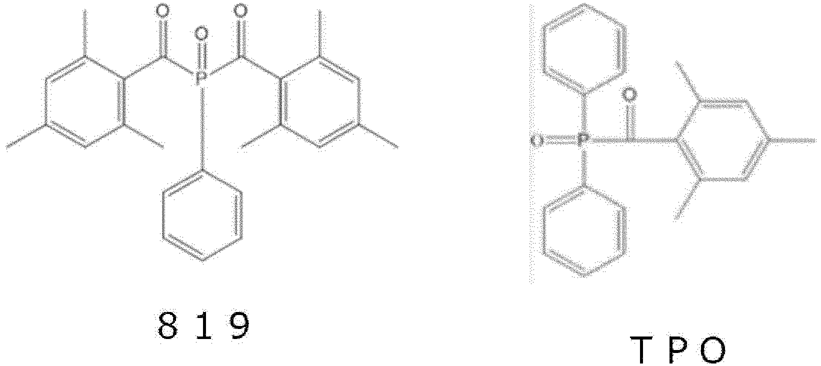

### [Producing Example 1A : production of SUA-1]

In a four-necked flask of 1 liter equipped with a well-dried stirring blade and a thermometer, 372 g (3.20 mol) of HEA (2-hydroxyethyl acrylate), 0.71 g of DBTDL (dibutyltin dilaurate, 0.1% by mass with respect to the total mass of HEA and TMHDI), and 0.35 g of MEHQ (4-methoxyphenol, 0.05% by mass of the total mass of HEA and TMHDI) were added and stirred until the mixture was homogeneous, and then the temperature was raised to 60°C. Subsequently, 337 g of TMHDI (trimethylhexamethylene diisocyanate, 1.60 mol) was added dropwise over 1 hour. As the internal temperature rose due to the reaction heat during the drop, the drop rate was controlled so that the temperature was below 80°C. After completion of the dropping of the total amount, the reaction was carried out for 10 hours while keeping the temperature at 80°C. At this time, the proceedings of reaction were tracked by HPLC analysis to confirm the endpoint of the reaction. The product was discharged from the reactor to obtain 680 g of a bifunctional urethane acrylate (SUA-1). The viscosity at 25°C was 7,100 mPa·s.

### [Producing Example 1B : production of SUA-2]

In a four-necked flask of 1 liter equipped with a well-dried stirring blade and a thermometer, 416 g (3.20 mol) of HEA (2-hydroxyethyl methacrylate), 0.75 g of DBTDL (0.1% by mass with respect to the total mass of HEA and TMHDI), and 0.38 g of MEHQ (0.05% by mass of the total mass of HEA and TMHDI) were added and stirred until the mixture was homogeneous, and then the temperature was raised to 60°C. Subsequently, 337 g (1.60 mol) of TMHDI was added dropwise over 1 hour. As the internal temperature rose due to the reaction heat during the drop, the drop rate was controlled so that the temperature was below 80°C. After completion of the dropping of the total amount, the reaction was carried out for 10 hours while keeping the temperature at 80°C. At this time, the proceedings of reaction were tracked by HPLC analysis to confirm the endpoint of the reaction. The product was discharged from the reactor to obtain 720 g of a bifunctional urethane acrylate (SUA-2). The viscosity at 25°C was 8,200 mPa·s.

### [Producing Example 1B : production of SUA-3]

In a four-necked flask of 1 liter equipped with a well-dried stirring blade and a thermometer, 222 g (1.00 mol) of IPDI (isophorone diisocyanate), 0.84 g of DBTDL (0.1% by mass with respect to the total mass of IPDI, PEG-1000 and TMHDI), and 0.42 g of MEHQ (0.05% by mass of the total mass of IPDI, PEG-1000 and TMHDI) were added and stirred until the mixture was homogeneous, and then the temperature was raised to 60°C. Subsequently, 500 g (0.50 mol) of PTMG1000 (molecular weight 1000, manufactured by Mitsubishi Chemical Corporation) was added dropwise over 1 hour. As the internal temperature rose due to the reaction heat during the drop, the drop rate was controlled so that the temperature was below 80°C. After completion of the dropping of the total amount, the reaction was carried out for 5 hours while keeping the temperature at 80°C.

Subsequently, the internal temperature of the flask was maintained at 60°C, and116 g (1.00 mol) of HEA added to a separate dropping funnel was added dropwise over 1 hour. As the internal temperature rose due to the reaction heat during the drop, the drop rate was controlled so that the temperature was below 80°C. After completion of the dropping of the total amount, the reaction was carried out for 5 hours while keeping the temperature at 80°C. At this time, the proceedings of reaction were tracked by HPLC analysis to confirm the endpoint of the reaction. The product was discharged from the reactor to obtain 840 g of a urethane acrylate (SUA-3). The viscosity at 40°C was 41,000 mPa·s.

As shown in Tables 2 and 3, in Examples, cured products with excellent deformability and shape recovery properties were obtained.

On the other hand, the cured products obtained in Comparative Examples 1 and 2 were easily deformed, and the cured product obtained in Comparative Example 3 did not recover its shape.

### [Example 20]

### <Production of plate denture>

A plaster model of the upper or lower jaw was used to make a 3D impression data using a laboratory dental scanner (Kulzer GmbH, Cara Scan 4.0). The 3D impression data was uploaded to commercially available CAD software (manufactured by 3D Systems, Geomagic Design X). A mold for producing a plate denture was designed using the CAD software, the thickness of the mold was set to 2.0 mm, and 3D modeling data was obtained.

The photocurable composition in Example 1 was irradiated with visible light having a wavelength of 405 nm at an irradiation dose of 11 mJ/cm² to form a cured layer with a thickness of 50 µm, and the cured layer was stacked in a thickness direction thereof and was modeled using the 3D modeling data of a mold which was obtained above to obtain a mold modeling product for the production of a plate denture. The obtained mold modeling product was irradiated with ultraviolet rays having a wavelength of 365 nm at an irradiation dose of 3 J/cm² and main cured to obtain a mold for the production of a plate denture.

Artificial teeth were arranged in the mold for producing a plate denture obtained above, a (meth)acrylate polymer powder for plate denture production and (meth)acrylate monomer liquid (Palapress (registered trademark), Vario manufactured by Kulzer GmbH) were mixed in a predetermined ratio, and the mixture was injected into the mold in which the artificial teeth were arranged. Then, it was covered with a plaster model, and polymerized at 55°C and 2 bar pressure for 30 minutes. After polymerization, the mold and plaster model were removed to obtain a plate denture. At this time, it was confirmed that neither the mold nor the plate denture was damaged.

Furthermore, this method makes it easier to obtain a plate denture than conventional manual methods using wax dentures or silicone, and because a denture resin that is unsuitable for photomodeling can be used to produce a plate denture, it is suitable for obtaining the desired physical properties.

### [Examples 20 to 43, Comparative Examples 4 to 8]

### <Preparation of photocurable compositions>

The components shown in Tables 4 to 7 were mixed to obtain photocurable compositions. Table 4 shows the details of each component, and Tables 5 to 7 show the mixing ratios of each component.

### <Measurement and evaluation>

The following measurements and evaluations were performed using the obtained photocurable compositions. The measurement and evaluation methods for physical properties, or the like, already explained, are omitted.

The results are shown in Tables 5 to 7.

### (Releasability for denture)

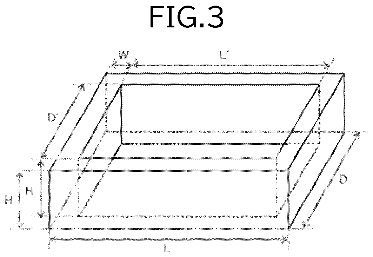

A test piece A4 shown in FIG. 3 was produced by photomodeling under conditions in which each of the obtained photocurable composition was irradiated with visible light having a wavelength of 405 nm at an irradiation dose of 11 mJ/cm² to form a cured layer A1 with a thickness of 50 µm, the cured layer A1 was stacked in a thickness direction thereof to form a three-dimensional modeling product A4 shown in FIG.3, and the modeling product A4 was irradiated with ultraviolet rays having a wavelength of 365 nm at an irradiation dose of 3 J/cm² to produce the test piece A4.

As shown in FIG. 3, the test piece A4 has a shape in which L is 24 mm, L' is 20 mm, W is 2 mm, H is 5 mm, H' is 3 mm, D is 14 mm, and D' is 10 mm. A (meth)acrylate polymer powder for plate denture production and Palapress (registered trademark) Vario (manufactured by Kulzer GmbH), which was (meth)acrylate monomer liquid, were mixed in a ratio of 10 g powder: 7 mL liquid, 15 seconds after mixing, the mixture was filled into the space of the test piece A4 having dimensions L' (20 mm) x D' (10 mm) x H' (3 mm), and polymerized at 55°C and 2 bar pressure for 30 minutes. Then, the test piece A4 was removed from the polymer for producing a plate denture by peeling it off. Thereafter, the surface of the polymer for producing a plate denture that had been in contact with the test piece A4 was observed using a 3D shape measuring device (VR-3200, manufactured by Keyence Corporation), and the area value of the test piece A4 that had adhered to the polymer for producing a plate denture after removal was calculated, and the adhesion rate relative to the surface (200 mm²) consisting of L' and D' was calculated. The smaller the adhesion rate, the better the releasability. The evaluation was performed as "A" if there was no surface adhesion, "B" if the surface adhesion was less than 5%, and "C" if the surface adhesion was 5% or more.

### (Toughness when demolding (ease of removal))

A test piece A4 shown in FIG. 3 was produced by photomodeling under conditions in which each of the obtained photocurable composition was irradiated with visible light having a wavelength of 405 nm at an irradiation dose of 11 mJ/cm² to form a cured layer A1 with a thickness of 50 µm, the cured layer A1 was stacked in a thickness direction thereof to form a three-dimensional modeling product A4 shown in FIG.3, and the modeling product A4 was irradiated with ultraviolet rays having a wavelength of 365 nm at an irradiation dose of 3 J/cm² to produce the test piece A4.

As shown in FIG. 3, the test piece A4 has a shape in which L is 24 mm, L' is 20 mm, W is 2 mm, H is 5 mm, H' is 3 mm, D is 14 mm, and D' is 10 mm. A (meth)acrylate polymer powder for plate denture production and Palapress (registered trademark) Vario (manufactured by Kulzer GmbH), which was (meth)acrylate monomer liquid, were mixed in a ratio of 10 g powder: 7 mL liquid, 15 seconds after mixing, the mixture was filled into the space of the test piece A4 having dimensions L' (20 mm) x D' (10 mm) x H' (3 mm), and polymerized at 55°C and 2 bar pressure for 30 minutes. Then, the test piece A4 was removed from the polymer for producing a plate denture by peeling it off. After removal, the appearance of the test piece A4 was observed and evaluated as "A" if there was no breakage and "B" if there was a breakage.

### (Shape recovery speed)

The obtained photocurable composition was modeled to the size of 8 mm length x 39 mm width x 4 mm thickness under conditions of visible light wavelength 405 nm and visible light illuminance 8.0 mJ/cm² using a 3D printer (Kulzer GmbH, Cara Print 4.0) to obtain a modeling product (layer width 50 µm).

The obtained modeling product was irradiated with ultraviolet light of a wavelength of 365 nm at 10 J/cm² for main curing of the modeling product to obtain a photofabrication product.

The obtained photofabrication product (hereafter referred to as "test piece") was curved by applying stress so that both ends of the test piece in the longitudinal direction (horizontal direction) were in contact with each other, and the test piece was held in that state for 10 seconds. The stress was then released and the change in shape of the test piece was observed and evaluated according to the following criteria:
A: After the stress was released, the test piece returned to its original shape within 1 second
B: After the stress was released, it took more than 1 second to return to its original shape.

**[Table 4]**

| | Component | Name | Mw | O-O interatomic distance d1(Å) | Number of functional group | Number of aromatic ring in monomer | Number of urethane bond in monomer | Number of siloxane bond in monomer |
|---|---|---|---|---|---|---|---|---|
| Composition | Mono(meth)acrylic monomer (X) | PO-A | 192.21 | - | 1 | 1 | 0 | 0 |
| | | PO | 206.24 | - | 1 | 1 | 0 | 0 |
| | | P2H-A | 236.27 | - | 1 | 1 | 0 | 0 |
| | | M-600A | 222.24 | - | 1 | 1 | 0 | 0 |
| | | POB-A | 254.29 | - | 1 | 2 | 0 | 0 |
| | | A-LEN-10 | 268.31 | - | 1 | 2 | 0 | 0 |
| | Di(meth)acrylic monomer (Y) | UDA | 442.51 | 14.8 | 2 | 0 | 2 | 0 |
| | | ABE-300 | 468.55 | 16.5 | 2 | 1 | 0 | 0 |
| | | A-BPE-10 | 776.92 | 33.7 | 2 | 2 | 0 | 0 |
| | | SA-001 | 446.5 | 15.3 | 2 | 1 | 0 | 0 |
| | | AH-600 | 612.68 | 14.4 | 2 | 2 | 2 | 0 |
| | | SA-002 | 951.16 | 46.3 | 2 | 0 | 2 | 0 |
| | | UA1 | 1632.21 | 96.3 | 2 | 0 | 4 | 0 |
| | Polyfunctional | SiA-001 | 432.74 | 13.3 | 2 | 0 | 0 | 3 |
| | (meth)acrylic monomer (Z) | SiA-002 | 1656.66 | 41.4 | 2 | 0 | 0 | 8.4 |
| | | SiA-003 | 1693.35 | 35.6 | 2 | 0 | 0 | 20 |
| | Other monomers | LA | 240.39 | - | 1 | 0 | 0 | 0 |
| | | 9EG-A | 522.59 | 31.6 | 2 | 0 | 0 | 0 |
| | Photopolymerization initiator | Omnirad819 | 348.38 | - | - | - | - | - |

**[Table 5]**

| | Component | Name | Comparative Example | | | | | Example | | | |
|---|---|---|---|---|---|---|---|---|---|---|---|
| | | | 4 | 5 | 6 | 7 | 8 | 20 | 21 | 22 | 23 |
| Composition | Mono(meth)acrylic monomer (X) | PO-A | | 90 | | | | 70 | 70 | 70 | |
| | | PO | | | | | | | | | 70 |
| | | P2H-A | | | | | | | | | |
| | | M-600A | | | | | | | | | |
| | | POB-A | | | | | | | | | |
| | | A-LEN-10 | | | | 90 | | | | | |
| | Di(meth)acrylic monomer (Y) | UDA | | | | | | 20 | 20 | 20 | 20 |
| | | ABE-300 | | | 70 | | 60 | | | | |
| | | A-BPE-10 | | | | | | | | | |
| | | SA-001 | | | | | | | | | |
| | | AH-600 | | | | | | | | | |
| | | SA-002 | | | | | 40 | | | | |
| | | UA1 | | | | | | | | | |
| | Polyfunctional (meth)acrylic monomer (Z) | SiA-001 | | | 30 | | | 10 | | | |
| | | SiA-002 | | | | 10 | | | 10 | | |
| | | SiA-003 | | | | | | | | 10 | 10 |
| | Other polymers | LA | 60 | | | | | | | | |
| | | 9EG-A | 40 | 10 | | | | | | | |
| | Photopolymerization initiator | Omnirad819 | 1 | 1 | 1 | 1 | 1 | 1 | 1 | 1 | 1 |
| Total | | | 101 | 101 | 101 | 101 | 101 | 101 | 101 | 101 | 101 |
| Siloxane bond concentration in composition (mmol/g) | | | 0.000 | 0.000 | 2.059 | 0.502 | 0.000 | 0.686 | 0.502 | 1.169 | 1.169 |
| Aromatic ring concentration in all monomers (mol/g) | | | 0.0000 | 0.0046 | 0.0000 | 0.0066 | 0.0000 | 0.0036 | 0.0036 | 0.0036 | 0.0034 |
| Releasability for dentureA:no surface adhesion, B:surface adhesion<5%, C:surface adhesion ≧ 5% | | | C | C | C | A | C | A | A | A | B |
| Toughness when demolding (ease of removal)@23°C A:no breakage, B:breakage | | | B | B | B | A | C | A | A | A | A |
| Storage elastic modulus at 25°C (MPa) | | | 6 | 7 | 1733 | 6 | 2169 | 43 | 32 | 33 | 48 |
| Storage elastic modulus at 37°C (MPa) | | | 4 | 6 | 763 | 5 | 1253 | 35 | 29 | 31 | 44 |
| Urethane bond concentration in all monomers (mol/g) | 0 | 0 | 0 | 0 | 0.00083 | 0.00089 | 0.00089 | 0.00089 | | 0.00089 | |
| Deformability A: <0.05mm, B : 0.05mm~0.10mm, C : >0.10mm | C | C | B | C | B | A | A | A | | A | |
| Shape recovery in removal test at 37°C A : no change in shape and no cracks, B : change in shape, C : crack | C | B | C | B | C | A | A | A | | A | |
| Shape recovery speed A : returned to its original shape within 1 second, B : took more than 1 second to return to its original shape | - | - | - | - | - | - | - | - | | - | |

**[Table 6]**

| | Component | Name | Example | | | | | | | | | |
|---|---|---|---|---|---|---|---|---|---|---|---|---|
| | | | 24 | 25 | 26 | 27 | 28 | 29 | 30 | 31 | 32 | 33 |
| Compositio n | Mono(meth)acrylic monomer (X) | PO-A | | | | | | | | | | |
| | | PO | | | | | | | | | | |
| | | P2H-A | 70 | | | | | | | | | |
| | | M-600A | | 70 | | | | | | | | |
| | | POB-A | | | 70 | | 70 | 70 | 70 | 70 | 70 | 70 |
| | | A-LEN-10 | | | | 70 | | | | | | |
| | Di(meth)acrylic monomer (Y) | UDA | 20 | 20 | 20 | 20 | | | | | | |
| | | ABE-300 | | | | | | | | | | |
| | | A-BPE-10 | | | | | 20 | | | | | |
| | | SA-001 | | | | | | 20 | | | | 10 |
| | | AH-600 | | | | | | | 20 | | | |
| | | SA-002 | | | | | | | | 20 | | 10 |
| | | UA1 | | | | | | | | | 20 | |
| | Polyfunctional (meth)acrylic monomer (Z) | SiA-001 | | | | | | | | | | |
| | | SiA-002 | 10 | 10 | | | | 10 | 10 | 10 | 10 | 10 |
| | | SiA-003 | | | 10 | 10 | 10 | | | | | |
| | Other polymers | LA | | | | | | | | | | |
| | | 9EG-A | | | | | | | | | | |
| | Photopolymerizatio n initiator | Omnirad81 9 | 1 | 1 | 1 | 1 | 1 | 1 | 1 | 1 | 1 | 1 |
| Total | | | 101 | 101 | 101 | 101 | 101 | 101 | 101 | 101 | 101 | 101 |
| Siloxane bond concentration in composition (mmol/g) | | | 0.502 | 0.502 | 1.169 | 1.169 | 1.169 | 0.502 | 0.502 | 0.502 | 0.502 | 0.502 |
| Aromatic ring concentration in all monomers (mol/g) | | | 0.0029 | 0.0031 | 0.0055 | 0.0052 | 0.0060 | 0.0059 | 0.0061 | 0.0055 | 0.0055 | 0.0057 |
| Releasability for denture A:no surface adhesion, B:surface adhesion<5%, C:surface adhesion ≧ 5% | A | A | A | A | A | A | A | A | A | | A | |
| Toughness when demolding (ease of removal)@23°C A:no breakage, B:breakage | A | A | A | A | A | A | A | A | A | | A | |
| Storage elastic modulus at 25°C (MPa) | 33 | 93 | 27 | 65 | 18 | 25 | 29 | 18 | 11 | | 21 | |
| Storage elastic modulus at 37°C (MPa) | 21 | 78 | 18 | 48 | 11 | 21 | 23 | 16 | 8 | | 17 | |
| Urethane bond concentration in all monomers (mol/g) | 0.00089 | 0.00089 | 0.00089 | 0.00089 | 0.00000 | 0.00000 | 0.00065 | 0.00042 | 0.00049 | | 0.00021 | |
| Deformability A: <0.05mm, B : 0.05mm~0.10mm, C : >0.10mm | A | A | A | A | A | A | A | A | A | | A | |
| Shape recovery in removal test at 37° C A : no change in shape and no cracks, B : change in shape, C : crack | A | A | A | A | A | A | A | A | A | | A | |
| Shape recovery speed A : returned to its original shape within 1 second, B : took more than 1 second to return to its original shape | - | - | - | - | B | B | B | B | B | | A | |

**[Table 7]**

| | Component | Name | Example | | | | | | | | | |
|---|---|---|---|---|---|---|---|---|---|---|---|---|
| | | | 34 | 35 | 36 | 37 | 38 | 39 | 40 | 41 | 42 | 43 |
| Compositio n | Mono(meth)acrylic monomer (X) | PO-A | | 60 | | | | | 20 | | 30 | |
| | | PO | | | | | | | | | | |
| | | P2H-A | | | | | | 30 | | 20 | | |
| | | M-600A | | | | | | | 30 | | | |
| | | POB-A | | | 70 | 60 | 40 | 30 | | 30 | | 60 |
| | | A-LEN-10 | 60 | | | | | | | | 30 | |
| | Di(meth)acrylic monomer (Y) | UDA | | 10 | 20 | 20 | 20 | | | | 10 | |
| | | ABE-300 | | | | | | | | | | |
| | | A-BPE-10 | 20 | | | | | | | | | |
| | | SA-001 | | | | | | 20 | | 20 | | |
| | | AH-600 | 10 | 20 | | | | | 40 | | 20 | |
| | | SA-002 | | | | | | | | 20 | | 10 |
| | | UA1 | | | | | | | | | | |
| | Polyfunctional (meth)acrylic monomer (Z) | SiA-001 | | | | | | | | | | 10 |
| | | SiA-002 | | | 10 | 20 | 40 | 20 | 10 | 10 | | 20 |
| | | SiA-003 | 10 | 10 | | | | | | | 10 | |
| | Other polymers | LA | | | | | | | | | | |
| | | 9EG-A | | | | | | | | | | |
| | Photopolymerizatio n initiator | Omnirad81 9 | 1 | 1 | 1 | 1 | 1 | 1 | 1 | 1 | 1 | 1 |
| Total | | | 101 | 101 | 101 | 101 | 101 | 101 | 101 | 101 | 101 | 101 |
| Siloxane bond concentration in composition (mmol/g) | | | 1.169 | 1.169 | 0.502 | 1.004 | 2.008 | 1.004 | 0.502 | 0.502 | 1.169 | 1.690 |
| Aromatic ring concentration in all monomers (mol/g) | | | 0.0053 | 0.0037 | 0.0055 | 0.0047 | 0.0031 | 0.0040 | 0.0037 | 0.0036 | 0.0044 | 0.0047 |
| Releasability for denture A:no surface adhesion, B:surface adhesion<5%, C:surface adhesion ≧ 5% | | | A | A | A | A | A | A | A | A | A | A |
| Toughness when demolding (ease of removal)@23 °C A:no breakage, B:breakage | | | A | A | A | A | A | A | A | A | A | A |
| Storage elastic modulus at 25°C (MPa) | | | 34 | 31 | 29 | 35 | 42 | 24 | 54 | 15 | 42 | 21 |
| Storage elastic modulus at 37°C (MPa) | | | 28 | 30 | 23 | 30 | 34 | 21 | 38 | 12 | 28 | 19 |
| Urethane bond concentration in all monomers (mol/g) | | | 0.00032 | 0.00109 | 0.00089 | 0.00089 | 0.00089 | 0.00000 | 0.00129 | 0.00042 | 0.00109 | 0.00021 |
| Deformability A: <0.05mm, B : 0.05mm~0.10mm, C : >0.10mm | | | A | A | A | A | A | A | A | A | A | A |
| Shape recovery in removal test at 37° C A : no change in shape and no cracks, B : change in shape, C : crack | A | A | A | A | A | A | A | A | A | | A | |
| Shape recovery speed A : returned to its original shape within 1 second, B : took more than 1 second to return to its original shape | A | A | B | B | B | A | A | A | A | | - | |

In Tables 5 to 7, the numbers in the "Composition" column for each Example and Comparative Example mean parts by mass, and a blank space means that the corresponding component is not contained.

Details of each component in Tables 5 to 7 are as shown below.

### <Mono(meth)acrylic monomer (X)>

Each structure of the mono(meth)acrylic monomer (X) described in Tables 4 to 7 is as shown below.
PO-A Manufactured by kyoeisha Chemical Co., Ltd.
PO Manufactured by kyoeisha Chemical Co., Ltd.
P2H-A Manufactured by kyoeisha Chemical Co., Ltd.
M-600 Manufactured by kyoeisha Chemical Co., Ltd.
POB-A Manufactured by kyoeisha Chemical Co., Ltd.
A-LEN-10 Manufactured by SHIN-NAKAMURA CHEMICAL Co., Ltd.

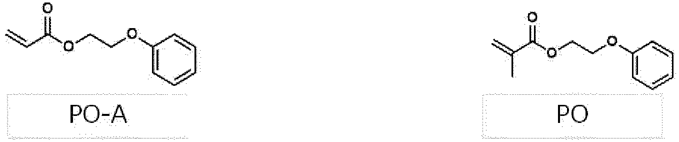

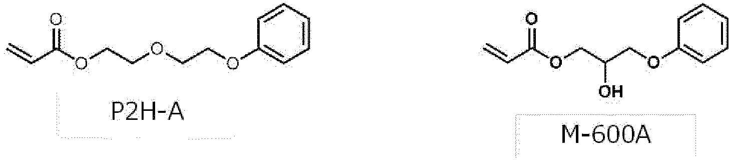

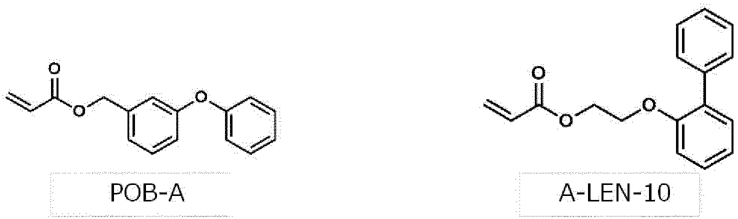

### <Di(meth)acrylic monomer (Y)>

Each structure of the di(meth)acrylic monomer (Y) described in Tables 4 to 7 is as shown below.
SUA-1 Compound produced by the method described in the following Producing Example 1A
(UDA in Table)
ABE-300 Manufactured by SHIN-NAKAMURA CHEMICAL Co., Ltd.
A-BPE-10 Manufactured by SHIN-NAKAMURA CHEMICAL Co., Ltd.
SA-001 Compound manufactured by the method described in the following Producing Example 2A
AH-600 Manufactured by kyoeisha Chemical Co., Ltd.
SA-002 Compound produced by the method described in the following Producing Example 2B
SUA-3 Compound produced by the method described in the following Producing Example 1C
(UA1 in Table)

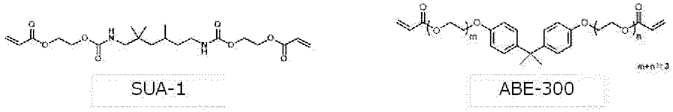

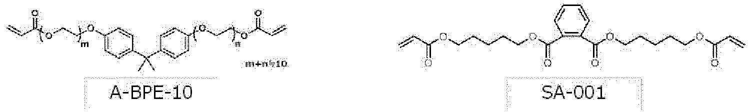

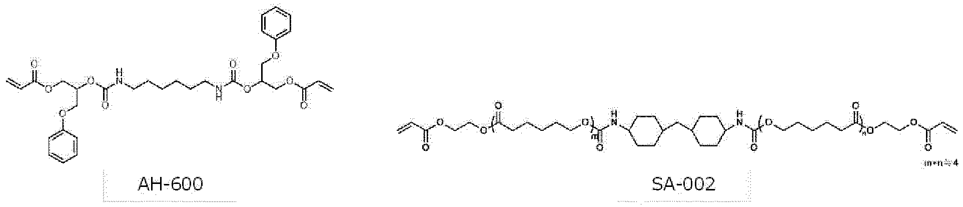

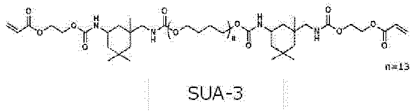

### [Producing Example 2A : production of SA-001]

In a four-necked flask of 2 liter equipped with a well-dried stirring blade and a thermometer, 170.81 g (1.64 mol) of pentanediol, phthalic anhydride 120.8 g (0.82 mol), p-toluenesulfonic acid 3.6 g, phenothiazine 0.5 g, and toluene 300 g were added, and the mixture was heated to 150 to 200°C in a nitrogen atmosphere and reacted for 5 hours. Subsequently, 122.50 g (1.70 mol) of acrylic acid was added, the mixture was further heated, and the reaction was carried out for 10 hours. The reaction product was dissolved in 500 g of toluene, neutralized with a 10% aqueous solution of NaOH, and then washed with 150 g of a 5% aqueous solution of ammonium sulfate. Toluene was distilled under reduced pressure to obtain 390 g of a di(meth)acrylic monomer (SA-001). The viscosity at 25°C was 410 mPa·s.

### [Producing Example 2B : production of SA-002]

In a four-necked flask equipped with a stirrer, an air inlet tube, and a thermometer, 344 g (1.00 mol) of caprolactone-modified 2-hydroxyethyl acrylate (product name "Placcel FA2D", manufactured by Daicel Corporation, average number of moles of caprolactone added: 2), 131 g (0.50 mol) of dicyclohexylmethane 4,4'-diisocyanate, 0.84 g of DBTDL (dibutyltin dilaurate), and 0.42 g of MEHQ (4-methoxyphenol) were added and the mixture was reacted at 80°C for 12 hours. At this time, the proceedings of reaction were tracked by HPLC analysis to confirm the endpoint of the reaction. The product was discharged from the reactor to obtain 451 g of a di(meth)acrylic monomer (SA-002). The viscosity at 25°C was 12,000 mPa·s.

### <Polyfunctional (meth)acrylic monomer (Z)>

Each structure of the polyfunctional (meth)acrylic monomer (Z) described in Tables 4 to 7 is as shown below.
SiA-001 Compound produced by the method described in the following Producing Example 2C
SiA-002 Compound produced by the method described in the following Producing Example 2D
SiA-003 Compound produced by the method described in the following Producing Example 2E

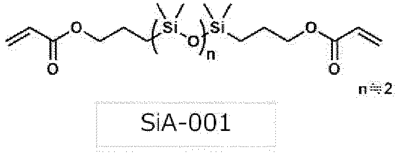

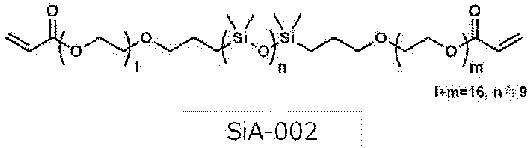

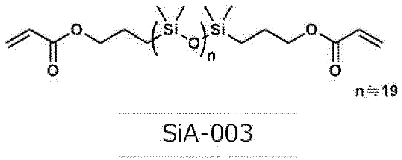

### [Producing Example 2C : production of SiA-001]

In a four-necked flask of 1 liter equipped with a well-dried stirring blade and a thermometer, 324 g (1.00 mol) of 3,3'-(1,1,3,3,5,5-Hexamethyl-1,5-trisiloxanediyl)bis[1-propanol], 0.30g of BHT 0.30g, and 500 g of ethyl acetate were added, and stirred until the mixture was homogeneous, and then the temperature was raised to 70°C. Subsequently, 181g (2.00 mol) of acryloyl chloride was added dropwise over 1 hour. As the internal temperature rose due to the reaction heat during the drop, the drop rate was controlled so that the temperature was below 70°C. After the entire amount was dropped, the reaction temperature was kept at 70°C and the reaction was carried out for 5 hours. Then the reaction solution was neutralized with a 10% aqueous solution of NaOH, and then washed with 150 g of a 5% aqueous solution of ammonium sulfate. Ethyl acetate was distilled under reduced pressure to obtain 440 g of a polyfunctional (meth)acrylic monomer (SiA-001). The viscosity at 25°C was 110 mPa·s.

### [Producing Example 2D : production of SiA-002]

In a four-necked flask of 1 liter equipped with a well-dried stirring blade and a thermometer, 287 g (1.00 mol) of 1,3-Bis(3-chloropropyl)-1,1,3,3-tetramethyldisiloxane, 0.30g of BHT 0.30g, and 500 g of toluene were added, and stirred until the mixture was homogeneous, and then the temperature was raised to 60°C. Subsequently, 593g (2.00 mol) of octamethylcyclotetrasiloxane was added dropwise over 1 hour. After the entire amount was dropped, the reaction temperature was kept at 60°C and the reaction was carried out for 6 hours. Subsequently, 740g (2.00 mol) of octaethylene glycol 740g (2.00 mol) was added to the reaction vessel and the reaction was carried out for 1 hours. Then 181g (2.00 mol) of acryloyl chloride was added dropwise over 1 hour. As the internal temperature rose due to the reaction heat during the drop, the drop rate was controlled so that the temperature was below 60°C. After completion of the dropping of the total amount, the reaction was carried out for 5 hours while keeping the temperature at 60°C. Then the reaction solution was neutralized with a 10% aqueous solution of NaOH, and then washed with 150 g of a 5% aqueous solution of ammonium sulfate. Toluene was distilled under reduced pressure to obtain 440 g of a polyfunctional (meth)acrylic monomer (SiA-003). The viscosity at 25°C was 310 mPa·s.

### [Producing Example 2E : production of SiA-003]

In a four-necked flask of 1 liter equipped with a well-dried stirring blade and a thermometer, 667 g (3.00 mol) of hexamethylcyclotrisiloxane, 100 mL of toluene, 200 mL of tetrahydrofuran, and 100 mL of hexane were added, and stirred until the mixture was homogeneous. Then, 2 mL of butyllithium (about 15% hexane solution) was added dropwise over 1 hour under ice cooling. After the entire amount was dropped, the reaction temperature was kept at room temperature and the reaction was carried out for 2 hours. Subsequently, 207 g (1.00 mol) of 3-(chlorodimethylsilyl)propyl acrylate added to a separate dropping funnel was added dropwise over 1 hour. After the entire amount was dropped, the reaction temperature was kept at 30°C and the reaction was carried out for 24 hours. At this time, the proceedings of reaction were tracked by HPLC analysis to confirm the endpoint of the reaction. Then the reaction solution was neutralized with a 10% aqueous solution of NaOH, and then washed with 150 g of a 5% aqueous solution of ammonium sulfate. The solvents were distilled under reduced pressure to obtain 812 g of a polyfunctional (meth)acrylic monomer (SiA-003). The viscosity at 25°C was 280 mPa·s.

### <Other monomers>

The structures of the other monomers described in Tables 4 to 7 are as follows: LA Manufactured by DKS Co. Ltd.
9EG-A Manufactured by DKS Co. Ltd.

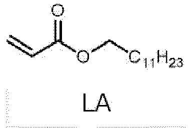

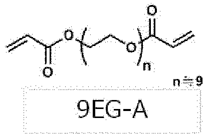

### <Photopolymerization initiator>

As the photopolymerization initiator shown in Tables 4 to 7, the aforementioned photopolymerization initiator 1 (acylphosphine oxide compound, Omnirad 819: "Omnirad 819" manufactured by IGM Resins B.V.) was used.

As shown in Tables 4 to 7, in Examples 20 to 43, cured products with excellent deformability and shape recovery were obtained.

On the other hand, the cured products obtained in Comparative Examples 4 to 8 were inferior in deformability and shape recovery effects compared to Examples 20 to 43.

Furthermore, in Examples 20 to 43, the evaluations of releasability and toughness were also good.

The present disclosures of Japanese Patent Application No. 2022-052228 filed on March 28, 2022 and Japanese Patent Application No. 2023-036051 filed on March 8, 2023 are hereby incorporated by reference in its entirety.

All the documents, patent applications, and technical standards that are described in the present specification are hereby incorporated by reference to the same extent as if each individual document, patent application, or technical standard is concretely and individually described to be incorporated by reference.

## Claims

1. A photocurable composition, comprising a (meth)acrylic monomer component, and a photopolymerization initiator, wherein:
in a case in which a rectangular sheet-like test piece A1 with a length of 40 mm, a width of 10 mm, and a thickness of 1.0 mm is produced by photomodeling under conditions in which the photocurable composition is irradiated with visible light having a wavelength of 405 nm at an irradiation dose of 11 mJ/cm² to form a cured layer A1 with a thickness of 50 µm, the cured layer A1 is stacked in a thickness direction thereof to form a rectangular sheet-like modeling product A1 with a length of 40 mm, a width of 10 mm, and a thickness of 1.0 mm, and the modeling product A1 is irradiated with ultraviolet rays having a wavelength of 365 nm at an irradiation dose of 3 J/cm² to produce the test piece A1,
a storage elastic modulus at 25°C of the test piece A1 is 10 MPa or more and
a storage elastic modulus at 37°C of the test piece A1 is 400 MPa or less.

2. The photocurable composition according to claim 1, wherein the (meth)acrylic monomer component comprises:
a mono(meth)acrylic monomer (X) having one (meth)acryloyloxy group and an aromatic ring,
a di(meth)acrylic monomer (Y) having at least one of a cyclic structure or a urethane bond, and two (meth)acryloyloxy groups and not having a siloxane bond, and
a polyfunctional (meth)acrylic monomer (Z) having a siloxane bond and two or more (meth)acryloyloxy groups.

3. A photocurable composition, comprising a (meth)acrylic monomer component, and a photopolymerization initiator, wherein the (meth)acrylic monomer component comprises:
a mono(meth)acrylic monomer (X) having one (meth)acryloyloxy group and an aromatic ring,
a di(meth)acrylic monomer (Y) having at least one of a cyclic structure or a urethane bond, and two (meth)acryloyloxy groups and not having a siloxane bond, and
a polyfunctional (meth)acrylic monomer (Z) having a siloxane bond and two or more (meth)acryloyloxy groups.

4. The photocurable composition according to claim 2 or claim 3, wherein a molecular weight of the di(meth)acrylic monomer (Y) is from 400 to 5000.

5. The photocurable composition according to claim 2 or claim 3, wherein a molecular weight of the polyfunctional (meth)acrylic monomer (Z) is from 400 to 5000.

6. The photocurable composition according to claim 2 or claim 3, wherein a content of the mono(meth)acrylic monomer (X) is from 30% by mass to 90% by mass with respect to a total amount of the (meth)acrylic monomer component.

7. The photocurable composition according to claim 2 or claim 3, wherein a content of the di(meth)acrylic monomer (Y) is from 5% by mass to 55% by mass with respect to a total amount of the (meth)acrylic monomer component.

8. The photocurable composition according to claim 2 or claim 3, wherein a content of the polyfunctional (meth)acrylic monomer (Z) is from 1% by mass to 60% by mass with respect to a total amount of the (meth)acrylic monomer component.

9. The photocurable composition according to claim 2 or claim 3, wherein a siloxane bond concentration in the composition is from 0.100 mmol/g to 3.000 mmol/g.

10. The photocurable composition according to claim 2 or claim 3, wherein an aromatic ring concentration in the (meth)acrylic monomer component is from 0.0015 mmol/g to 0.0070 mmol/g.

11. The photocurable composition according to claim 1, wherein the (meth)acrylic monomer component comprises di(meth)acrylic monomer (A) which has two (meth)acryloyloxy groups and an aromatic ring, and has a distance of from 25 Å to 80 Å between an oxygen atom forming an oxy group in one of the (meth)acryloyloxy groups and an oxygen atom forming an oxy group in another of the (meth)acryloyloxy groups.

12. The photocurable composition according to claim 1, satisfying either of the following (a) or (b):
(a) the (meth)acrylic monomer component comprises two or more types of di(meth)acrylic monomers (A) which have two (meth)acryloyloxy groups and an aromatic ring, and have a distance of from 25 Å to 80 Å between an oxygen atom forming an oxy group in one of the (meth)acryloyloxy groups and an oxygen atom forming an oxy group in another of the (meth)acryloyloxy groups,
(b) the (meth)acrylic monomer component comprises one or more (meth)acrylic monomer (B) selected from the group consisting of:
a di(meth)acrylic monomer (A) which has two (meth)acryloyloxy groups and an aromatic ring, and has a distance of from 25 Å to 80 Å between an oxygen atom forming an oxy group in one of the (meth)acryloyloxy groups and an oxygen atom forming an oxy group in another of the (meth)acryloyloxy groups;
a di(meth)acrylic monomer (B-1) which has two (meth)acryloyloxy groups and at least one of an aromatic ring or a urethane bond, and has a distance of 10 Å or more and less than 25 Å between an oxygen atom forming an oxy group in one of the (meth)acryloyloxy groups and an oxygen atom forming an oxy group in another of the (meth)acryloyloxy groups;
a di(meth)acrylic monomer (B-2) which has two (meth)acryloyloxy groups and at least one of an aromatic ring or a urethane bond, and has a distance of more than 80 Å and less than 200 Å between an oxygen atom forming an oxy group in one of the (meth)acryloyloxy groups and an oxygen atom forming an oxy group in another of the (meth)acryloyloxy groups; and
a mono(meth)acrylic monomer (B-3) which has one (meth)acryloyloxy group and at least one of an aromatic ring or a hydroxy group.

13. The photocurable composition according to any one of claims 1 to 3, having a viscosity, which is measured by an E-type viscometer under conditions of 25°C and 50 rpm, of from 5 mPa·s to 6,000 mPa·s.

14. The photocurable composition according to any one of claims 1 to 3, which is a photocurable composition for photomodeling.

15. The photocurable composition according to any one of claims 1 to 3, which is a photocurable composition used in production of a mold by photomodeling.

16. A three-dimensional modeling product, comprising a cured product of the photocurable composition according to any one of claims 1 to 3.

17. A mold, comprising the three-dimensional modeling product according to claim 16.

18. The mold according to claim 17, which is a mold used in production of a plate denture.

19. A method of producing a cured product, comprising a step of polymerizing a curable composition in the mold according to claim 17.

20. A method of producing a plate denture, comprising a step of curing a photocurable composition by photomodeling to produce a mold used in a production of a plate denture, and
a step of polymerizing a curable composition in the mold to produce a plate denture.
